(19) **Europäisches Patentamt / European Patent Office / Office européen des brevets**

(11) **EP 2 848 196 B1**

(12) # EUROPEAN PATENT SPECIFICATION

(45) Date of publication and mention
of the grant of the patent:
**27.06.2018 Bulletin 2018/26**

(51) Int Cl.:
**A61B 5/117** *(2016.01)* **A61B 5/00** *(2006.01)*

(21) Application number: **14178529.5**

(22) Date of filing: **25.07.2014**

(54) **Apparatus for blood vessel imaging**

Gerät zur Erzeugung von Aufnahmen der Blutgefässe

Appareil pour la photographie des vaisseaux sanguins

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.07.2013 JP 2013155158**

(43) Date of publication of application:
**18.03.2015 Bulletin 2015/12**

(73) Proprietor: **Hitachi, Ltd.**
**Tokyo 100-8280 (JP)**

(72) Inventors:
• **Miura, Naoto**
**Tokyo, 100-8280 (JP)**
• **Matsuda, Yusuke**
**Tokyo, 100-8280 (JP)**
• **Kiyomizu, Harumi**
**Tokyo, 100-8280 (JP)**

• **Nagamasa, Akio**
**Tokyo, 100-8280 (JP)**
• **Miyatake, Takafumi**
**Tokyo, 100-8280 (JP)**

(74) Representative: **MERH-IP Matias Erny Reichl Hoffmann**
**Patentanwälte PartG mbB**
**Paul-Heyse-Strasse 29**
**80336 München (DE)**

(56) References cited:
| | |
|---|---|
| **EP-A1- 1 832 231** | **EP-A2- 2 312 495** |
| **EP-A2- 2 450 829** | **WO-A1-2009/053920** |
| **US-A1- 2005 047 632** | **US-A1- 2008 049 982** |
| **US-A1- 2008 130 969** | **US-A1- 2011 063 430** |
| **US-A1- 2011 299 740** | **US-A1- 2013 182 921** |

**Description**

Technical Field

The present invention relates to a blood vessel image capturing apparatus as defined in claim 1.
Such an apparatus can be included in an authentication system for authenticating an individual using a living body, to provide a small, highly convenient, and highly accurate authentication technique.

Background Art

Among various biometric authentication techniques, finger veins authentication has been known as being capable of realizing highly accurate authentication. The finger vein authentication realizes superior authentication accuracy by using blood vessel patterns of the inner parts of fingers, and also realizes high level of security, because falsification and alteration are difficult in the finger authentication compared to fingerprint authentication.
Recently, portable terminals such as portable phones, notebook-type personal computers (PCs), and smartphones, and other devices including lockers, safety boxes, and printers which include a biometric authentication apparatus have been increased to assure security of each device. The biometric authentication can be applied in such fields as entry and exit management, attendance management, computer log-in, as well as recent application to payment of transactions. The biometric authentication apparatus particularly used in public scenes is required not only to authenticate individuals without fail, but also to increase a throughput of the apparatus. The throughput of the apparatus is affected by not only authentication speed and retry times, but also operation times of a user. Therefore, it is important to provide an authentication apparatus capable of performing highly accurate authentication and allowing anyone to operate the apparatus easily. Conditions to provide an easily operable and convenient authentication apparatus include, for example, an easy and intuitive way to use the apparatus, and smaller restrictions in presenting and placing a living body. Further, in view of recent popularity of tablet-type portable terminals and an increasing trend of wearable computing, a condition to realize a smaller apparatus while assuring convenience, as mentioned above, is also an important factor.
In PTL 1, a structure of an apparatus in which the apparatus is designed openly for the sake of increasing convenience of users, while providing a finger rest to place a finger thereon between the tip of the finger and a root of the finger in such a manner that the finger is not displaced from the position.
PTL2 discloses a finger detection device to detect a finger contour and vein information from a pressing degree.

Citation List

Patent Literature

PTL1: Japanese Patent Application Laid-open No. 2011-194245

PTL2: EP2312495

Summary of Invention

Technical Problem

In the course of miniaturization of the apparatus, however, there is a case where a structure such as a finger rest on which a finger of a user is placed has been removed, and the finger is presented on the apparatus in a complicated manner. Accordingly, it is not possible to obtain a desired blood vessel image for authentication, because a large positional deviation may occur at the spot where the image is taken, or a lot of noise such as blurs and wrinkles is projected in the obtained images to decrease authentication accuracy.
Therefore, an object of the present invention is to realize a blood vessel image capturing apparatus configured to obtain biometric feature information which includes a lot of information on personal features, as defined in claim 1. The blood vessel image capturing apparatus is small and convenient for use, and keeps a high degree of freedom in presenting a living body, in order to perform highly accurate personal authentication.

Solution to Problem

A blood vessel image capturing apparatus includes: a position area on which a finger is placed, a light source configured to emit a light to a finger presented on the position area, an imaging unit configured to form an image of light emitted from the light source and transmitted through the finger, a detecting unit with at least four pressure sensors provided at four location, configured to detect pressure of the finger placed on the position area, and an image processing unit configured to process an image formed by the imaging unit. The image processing unit obtains a position of a pressed region where the pressure of the finger is at or more than a predetermined pressure value, determines a pressure vector which is defined as having a direction towards the center of the pressed region, and that is determined according to a ratio of the pressure values of the four pressure sensors, calculates similarity between the pressure sensor of the pressed region and a registered pressure vector determined from each of previously registered plural images, and selects, according to the calculated similarity, a registered image of the registered plural images to be collated with the image: Advantageous Effects of Invention

A small, highly convenient, and highly accurate authentication apparatus using a finger, in which bend or flexing of a finger hardly occurs, and even when the finger is pressed, the authentication can still be performed with high accuracy, can be provided.

Brief Description of Drawings

[FIG. 1] FIG. 1 illustrates an entire structure of a biometric authentication system according to a first example.
[FIG. 2] FIG. 2 is an explanatory view illustrating a structure of the biometric authentication system with four pressure sensors according to the first embodiment of the invention.
[FIG. 3] FIG. 3 is a flowchart illustrating an example of authentication processing of the biometric authentication system according to the first example.
[FIG. 4] FIGS. 4A and 4B are explanatory views illustrating an example of overall processing of a wrinkle removing technique of the past.
[FIG. 5] FIG. 5 is an exemplary conceptual view illustrating an optical behavior model of the inner part of a living body.
[FIG. 6] FIGS. 6A to 6D are explanatory views illustrating processing procedures of removing wrinkles of the skin in the biometric authentication system according to the first example.
[FIG. 7] FIG. 7 illustrates an exemplary model of the inner side of the living body when the biometric authentication system according to the first example is used to measure a depth of blood vessels based on a two-wavelength transmitted image.
[FIG. 8] FIGS. 8A and 8B are explanatory views illustrating a principle of increasing a discriminating ability by using a depth of the blood vessel in the biometric authentication system according to the first example.
[FIG. 9] FIG. 9 is an explanatory diagram illustrating a relation between a change of pressure with time and timing of ending photographing in a biometric authentication system with four pressure sensors according to the first embodiment.
[FIG. 10] FIGS. 10A to 10C are explanatory views illustrating the concept and a calculating method of pressure vectors in the biometric authentication system according to the invention.
[FIG. 11] FIGS. 11A to 11B are explanatory views illustrating collation data using measured pressure and acquired finger vein patterns in the biometric authentication system according to the first example.
[FIG. 12] FIGS. 12A and 12C are explanatory views illustrating the process and result of collation processing using measured pressure and acquired finger vein patterns in the biometric authentication system according to the first example.
[FIG. 13] FIG. 13 illustrates an example in which light sources are arranged to surround a finger in the biometric authentication system according to the first example.
[FIG. 14] FIGS. 14A and 14B illustrate an example of the authentication apparatus in which light sources are arranged circularly below a finger in the biometric authentication system according to the first example.
[FIG. 15] FIGS. 15A to 15D illustrate an example of restructuring a blood vessel image that has been photographed by the authentication apparatus in which the light sources are arranged circularly below the finger in the biometric authentication system according to the first example.
[FIG. 16] FIG. 16 is an exemplary view illustrating the biometric authentication system according to the first example when installed on a portable terminal.
[FIG. 17] FIGS. 17A to 17C are explanatory views illustrating a structure of the apparatus of the biometric authentication system.
[FIG. 18] FIGS. 18A and 18B are exemplary views illustrating finger guidance displayed on the touch panel in the biometric authentication system.
[FIG. 19] FIG. 19 is an exemplary view illustrating a photographing state of a finger and a face of a user when reflected by the mirror and projected in the biometric authentication system.

Description of Embodiments and Examples

First Example

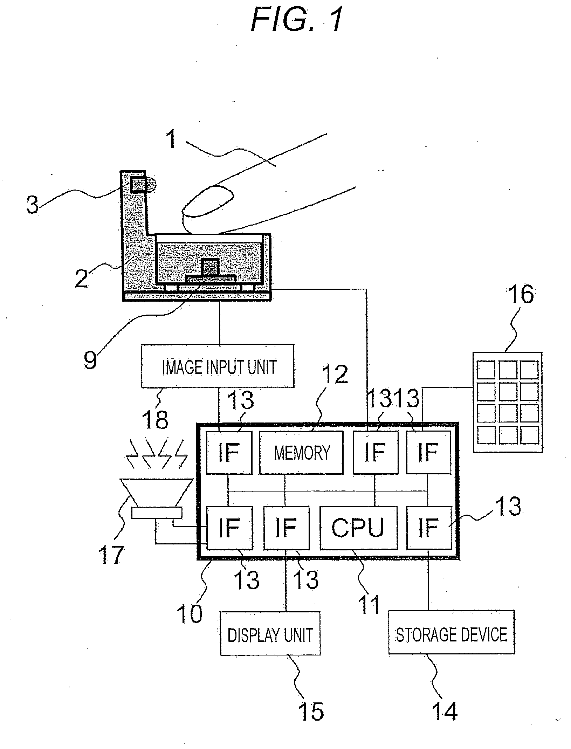

FIG. 1 illustrates an entire structure of a biometric authentication system according to a first example. The apparatus of the present example may be realized as a personal authentication apparatus including authentication processing. Alternatively, a blood vessel image capturing apparatus or a blood vessel image extracting apparatus may be specialized in acquiring blood vessel images and authentication processing may be performed external to the apparatus. An example of a terminal is also possible as will be described later.

The system of the first example includes an input device 2, an authentication processing unit 10, a storage device 14, a display unit 15, an input unit 16, a speaker 17, and an image input unit 18.

The input device 2 includes a housing in which a light source 3 is provided on the housing and an imaging device 9 is provided in the housing. A portion of the authentication processing unit 10 performing an image processing function, or such a portion and the image input unit 18 together may sometimes be referred to as the image processing unit. In any case, the authentication processing unit 10 has an image processing function.

The light source 3 is implemented as a light emitting element such as an infrared light emitting diode (LED) from which infrared light is emitted to a finger 1 presented on the input device 2. The imaging device 9 is configured to photograph an image of the finger 1 presented on the input device 2.

The image input unit 18 obtains an image photographed by the imaging device 9 of the input device 2 and inputs the obtained image to the authentication processing unit 10.

The authentication processing unit 10 includes a central processing unit (CPU) 11, a memory 12, and various other interfaces (IF) 13.

The CPU 11 executes programs stored in the memory 12 in order to perform various types of processing. The memory 12 stores a program to be executed by the CPU. The memory 12 also stores temporarily the image entered by the image input unit 18.

The interface 13 connects the authentication processing unit 10 with an external device. Specifically, the input device 2, the storage device 14, the display unit 15, the input unit 16, the speaker 17, the image input unit 18, etc. are connected to the interface 13.

The storage device 14 has pre-stored registration data of users. The registration data is, for example, a finger vein pattern image that is used to collate users. A typical finger vein pattern image is an image of a dark shadow pattern of blood vessels (finger veins) which are mostly distributed on the palm side of the skin of a finger.

The display unit 15 is, for example, a liquid display and serves as an output device configured to display the information received from the authentication processing unit 10.

The input unit 16 is, for example, a keyboard to transmit the information having been input by the user to the authentication processing unit 10. The speaker 17 is an output device which transmits the information received from the authentication processing unit 10 as an audio signal (e.g., voice).

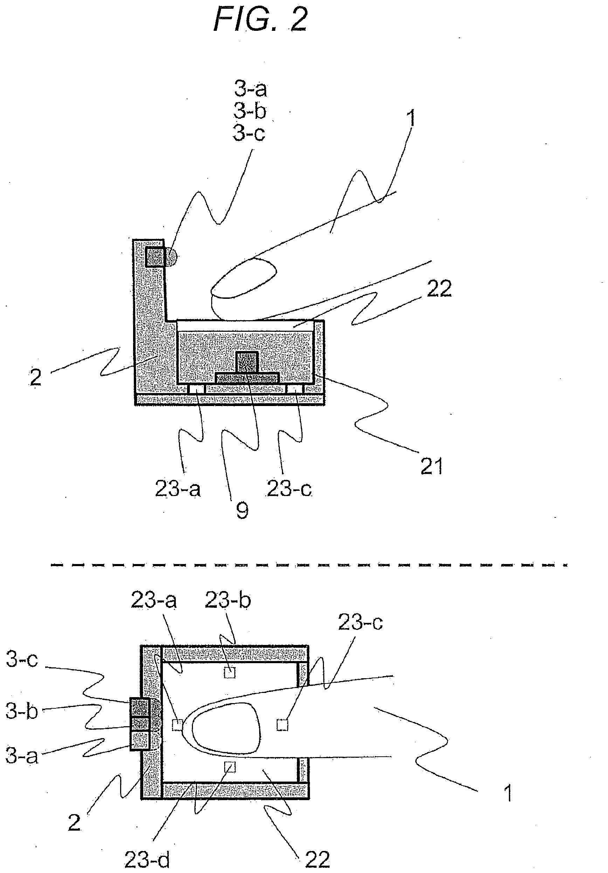

FIG. 2 is an explanatory view illustrating a structure of the input device of the biometric authentication system according to the first example.

The input device 2 is a personal authentication device including a position area where a fingertip is placed. The position area refers to an area where the finger is actually placed, and is formed by, for example, an acrylic plate 22 or a frame body around the acrylic plate 22 in the present example. This is, just an example, and it is sufficient to form such an area on the surface of the housing where a photographed image of the finger is presented, even when the finger is not in contact with the surface of the housing.

The light sources 3-a, 3-b, and 3-c are arranged in front of the finger 1 and emit light to the finger 1 from the front. In the present example, it is assumed that each light source can emit a different wavelength of light. A camera housing supporting unit 21 is formed in the apparatus, in which the acrylic plate 22 is fixed on the top surface of the camera housing supporting unit 21 to receive a fingertip, and a camera 9 is fixed below the acrylic plate 22. Pressure sensors 23 (23-a, 23-b, 23-c and 23-d) are provided under the camera housing supporting unit 21. The pressure sensors 23 and the camera housing supporting unit 21 are fixed with each other at contact points. The camera housing supporting unit 21 is integrated with the input device 2.

In the following description, it is assumed that the three light sources emit light of different wavelengths, but it may also be possible to provide a structure in which a single light source emits multiple types of light of different wavelengths.

When the finger 1 is pressed on the acrylic plate 22, the camera housing supporting unit 21 is pressed downwards accordingly to exert its pressure on the pressure sensor 23. As a result, the pressure of the finger 1 pressing down the acrylic plate 22 can be detected.

According to the invention, at least four pressure sensors 23 are provided at four locations relative to the center of the finger position area which is formed by the acrylic plate 22 and the like. The four locations refer to the fingertip side, the finger root side, and both right and left sides of the finger. The number of locations, however, where the pressure sensors 23 are provided is not limited to four, and the pressure sensors 23 may be placed at eight locations instead of four locations.

Further, the pressure sensors have been used in the present invention. Also, any other pressure detecting unit capable of detecting pressures can be used, other than sensors, so long as such a portion can detect pressures.

A balance of pressure in both right and left directions and front and back directions can be measured while the finger is pressing down on the acrylic plate 22. When the user presents the finger 1 on the acrylic plate 22, an electric characteristic of the pressure sensors 23 changes according to the intensity of pressing by the finger and, in response to the change, a pressure signal is output and supplied to the authentication processing unit 10 via the interface 13. It is assumed that tare weight of the acrylic plate 22 and the housing of the apparatus have been previously calibrated and are not considered as part of the pressure data.

The pressure sensors 23 can be arranged between the acrylic plate 22 and the camera housing supporting unit 21 to obtain a substantially similar effect. In this case, however, since the pressure sensors may accompany a little bit of deformation due to the pressure, it is expected that a positional relation between the optical axis of the camera 9 and the acrylic plate 22 is slightly changed. Meanwhile, such an effect can be alleviated by arranging the pressure sensors on the inner bottom of the housing, as in the structure of the present example. In addition, since a substrate that controls the camera, the light sources, etc. is often arranged intensively in the lower part of the apparatus, implementation of electric wirings of the pressure sensor would be easier when the pressure sensors are provided in the lower part of the apparatus.

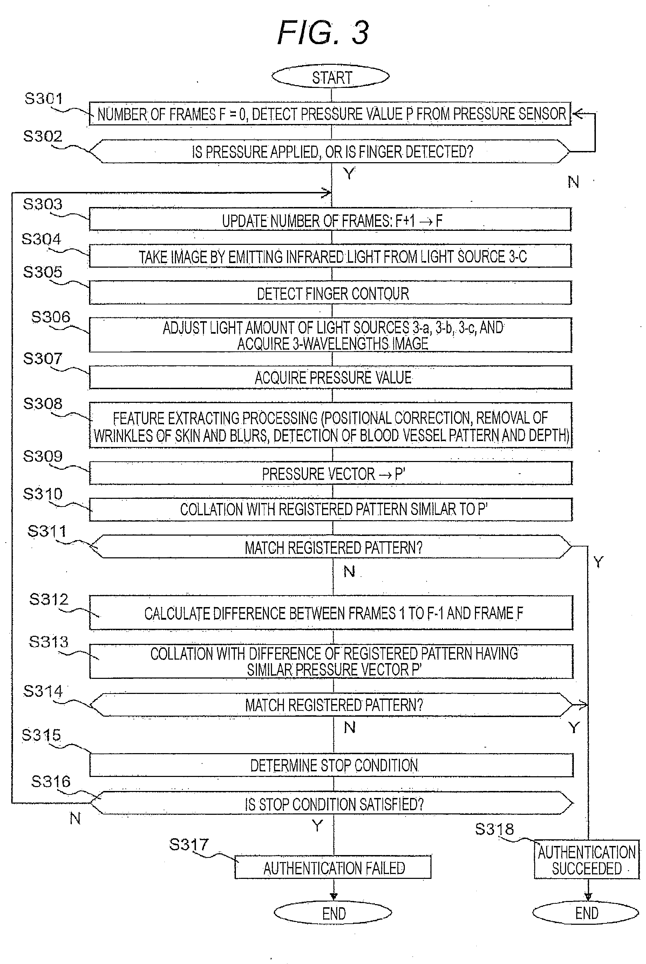

FIG. 3 is a flowchart illustrating an example. The present example will be described in detail by using the processing flow.

First, processing of reading a pressure value P from the pressure sensors is performed (S301). Since no image has been taken at this stage, initializing processing of retuning the number of frames F, which represents the photographed number of frames, to zero is also performed. The strength of the pressure of the finger is obtained by calculating a sum of pressure values of all of, for example, four pressure sensors. At this time, it is determined whether the pressure signal output from the pressure sensors exceeds a certain threshold value, or the presence of the finger is determined by a different method (S302). If no pressure is detected or it is determined that the finger is absent, the process returns to the last step. If the presence of the finger is detected, the process proceeds to the next step to start authentication processing.

As a method for determining the presence of the finger, other than detecting the pressure, a method capable of detecting fingers even when the finger is placed so lightly that the pressure sensor cannot respond is preferable. As an example of such a method, an image is photographed while any one of the light sources 3-a, 3-b, and 3-c is made to flash. According to the flashing cycle, if a change of luminance is observed in more than a fixed area, the presence of the finger on the apparatus can be determined.

By using this method with the method for detecting the pressure, the authentication starts when a fixed amount of pressure is exerted, or when a subject having more than a fixed area is held above the apparatus. Accordingly, the authentication processing can start in a stable manner. Meanwhile, during the detection of the pressure, a balance of output values from individual pressure sensors may be checked. If the pressure is significantly biased, the authentication does not start, and which fact is displayed to a user to guide the user to a correct position. For example, when a large pressure is applied to the pressure sensor 23-c alone that is located in the front of the apparatus, an instruction is given to move the finger towards the front side of the apparatus. Meanwhile, if the total pressure is at or more than a certain level, it is determined that the finger is pressed too much and the user may be guided to loosen the pressure. By doing this, the finger can be guided to a correct position and a correct pressure state.

When the finger is presented, the number of frames F to be photographed is increased by one (S303), and the light source 3-c is turned on to obtain light to transmit through the finger (S304). It is assumed, however, the light source 3-c has the weakest light spectrum components of ambient light compared to the wavelengths of the other two light sources. Accordingly, the subsequent processing such as a finger contour detection, which will be described later, becomes robust to the influence of outside light. The light source emits light to the back side of the finger 1. The light is scattered inside the finger and output from the skin of the finger 1 located on the palm side and reaches to the camera 9. At this time, a brightly shining image of the finger 1 is photographed, and an approximate position of the finger 1 can be known. A light quantity value of the emitted light can be set to a value sufficient to obtain an average luminance value with an average finger. Alternatively, when a 1 : 1 authentication is performed on the premise of inputting an ID, an optimal light quantity which has been determined and saved at the time of registration may be emitted. This is advantageous in that a light quantity close to the optimal value can be emitted without performing light quantity control processing which will be described later.

Next, a finger contour is detected according to the obtained image (S305). The finger 1 shines brightly by the irradiation of transmitted light, and the contour projected on the image can be detected. At this time, background of the image may also shine brightly. To separate the background from the finger region more accurately, the image of the finger is photographed without turning on the light source 3-c, and a difference between images taken with and without turning on the light source 3-c is determined. Thus, the finger contour detection robust to the influence of the outside light can be performed. The image of the finger photographed in this manner is then subjected to processing for acquiring coordinates of the contour. This is performed by using common contour detecting processing or finger area classification processing, such as a method using a common edge enhancement filter and edge connection of enhanced edges, a method combining statistical method such as random sample consensus (RANSAC) and a Kalman filter, or segmentation between the finger area and the background area according to a graph cut theory. A portion of the contour of the finger having the highest curvature is regarded as a fingertip. If the position of the fingertip cannot be observed, feedback is returned to the user to relocate the finger. Since the position of the fingertip is a reference position for positional correction, guiding the fingertip to be placed at a position where the fingertip can be shown clearly might increase the accuracy of positional correction in the subsequent collating processing, which leads to contribution to improvement of authentication accuracy.

Subsequently, adjustment of light amount from the light sources of three wavelengths is performed (S306). The contour of the finger detected by the above processing, and the light amount is adjusted such that an average luminance of the internal area of the contour is made to a constant value. Feedback control is performed, including decreasing the light amount when an average luminance value is high, and increasing the light amount value when the average luminance value is low. The amount of change of the light amount value can be controlled by formulating in advance a relation between the light amount value and the average luminance value, and emitting light of the light amount value at which a target average luminance value can be obtained. Accordingly, the high speed converging can be performed. The adjustment of the light amount is controlled independently for each wavelength of the light source. Since the brightness of an image changes for each wavelength, the light amount value may be controlled separately by feedback. If, however, a relation between the sensitivity of a camera for each wavelength and the luminance value of the light amount value having been set is previously known, and a proper light amount for a certain wavelength was given, then the light amount for other wavelengths can be estimated. If such a calibration is performed, no feedback control is needed for adjustment, and high speed photographing can be realized. The light sources of three wavelengths are subjected to this processing to obtain three transmitted images.

To measure the pressure of the fingertip corresponding to the currently obtained image, the current pressure sensor values are read (S307). Accordingly, it is known that in what degree of pressure the image has been photographed.

Next, feature extraction processing is performed using the obtained image (S308). The processing includes processing for correcting position and rotation of fingers according to the finger contour, wrinkle removing processing, blur removing processing, blood vessel pattern detecting processing, and processing for detecting the depth of blood vessels. The positional correction is performed as follows. The position of the fingertip of the finger contour is moved in parallel to a certain position of the image, while the longitudinal direction of the finger is determined from the finger contour and rotated in parallel with the horizontal direction of the image. Thus, an approximate position of the finger and a planar revolution of the finger can be normalized. Subsequently, processing for separating wrinkles formed on the surface of the finger from the image of blood vessels in the body, processing for removing blurs of the blood vessel image associated with diffusion of light, and processing for measuring the depth of the blood vessel are performed. Thus, clear and abundant information of the structure of the blood vessel can be obtained. The above processing will be described in detail later. From the processing result of the blood vessel image, a blood vessel pattern is extracted. The extraction can be performed using common feature extraction processing, including enhancement processing such as a Gabor filter, detection of a center axis of the blood vessel by calculating curvature of the cross-section of luminance, and processing for statistically connecting line information. As preprocessing of the extraction processing of the blood vessel, the blood vessel pattern may be enhanced by determining a difference between the images using a visual difference of the blood vessel in the image of each wavelength. This, however, generates a similar effect as the effect of the blood vessel image blur removing method, such that the calculation of the difference of the images may or may not be performed.

Subsequently, a pressure vector P' is obtained (S309). The pressure vector represents normalized pressure information that has been calculated using a relation between specific values of the pressure sensor and the contour position of the finger. A method for calculating the pressure vector will be described later.

Registered data that includes a pressure vector similar to the pressure vector P' is extracted from a database, and similarity with the input data is determined by collating the registered data with the input pattern (S310). To determine the similarity of patterns, a common method for determining similarity using template matching or minutiae matching, vector matching based on directional components of lines, or scale-invariant feature transform (SIFT), which represents an information amount of local luminance, histograms of oriented gradients (HOG), or local binary pattern(LBP) may be used.

Accordingly, the registration data can be narrowed by using the similarity of pressure vectors to determine candidate data. Using such candidate data, the similarity of the blood vessel pattern is determined, and high speed authentication processing in the 1 : N authentication is realized. The pressure vector largely depends on personal habits and is generated as information based on the pressed region of the photographed blood vessel image. Therefore, the pressure vector can also be used as effective information to improve the authentication accuracy even in a poor image quality, which will be described in detail later.

Next, according to the result of the previous step, it is determined whether statistical similarity is recognized between both patterns (S311) and, when the similarity is recognized, it is regarded that the finger is of one of the registered persons. The authentication ends as it is determined that the authentication has succeeded (S318).

If the patterns do not match, difference images are generated for all combinations of the current and past photographed patterns (S312), and the collation is similarly performed on the difference images that have been generated from all registered data including similar combinations of the current and past pressure vectors (S313). Then the similarity of both patterns is determined again (S314) and, if a match is found, it is regarded that the authentication has succeeded (S318) and the authentication ends. If the authentication failed, processing for determining a photographing stop condition (S315) to determine whether the stop condition is satisfied (S316). If the stop condition is not satisfied, the process returns to the imaging processing (S303). If the stop condition is satisfied, it is regarded that the authentication has failed, and the authentication system rejects the input finger (S317).

The stop condition of photographing the finger mentioned in step S315 is described. Four stop conditions are considered herein for determination. That is, not to stop until at least a predetermined number of frames of finger images are acquired, not to stop until the finger itself is sufficiently stopped, to stop when the pressure value is stable for a certain period of time, and to stop when the timeout is reached.

First, by setting the condition to acquire at least a predetermined number of frames of finger images, successive frames acquired can have a variation to improve the authentication accuracy. Next, by setting the condition to determine the stationary state of the finger, it is possible to guarantee illumination from the most suitable light source for the current finger position. In the present embodiment, however, because the contour of the finger has been detected, the positional deviation of the finger can be corrected through image processing, and the normalization such as moving the finger to the center position of the image can be performed. Thus, when the finger is located at a position apparently illuminated by the light from the light source, and there is no large deviation as a result of positional correction of the finger, it may be determined that the finger is stationary. By determining the positional deviation after the correction, it can be known that acquiring images having large variations is not expected even if the finger is moving in practice. This has an effect that the authentication can end earlier. Next, in the condition to stop when the pressing pressure of the finger is stabilized, various correlations can be obtained between the change of the finger image and the change of pressure while the image of the finger veins is changing by pressing the finger. However, when it is not expected to succeed in the authentication even after the pressure value is stabilized, it may be better to abandon the authentication for the sake of contribution to maintaining the throughput of the apparatus. When the timeout is reached, even though the provided photographing conditions are insufficient, the maximum response time can be fixed and at least a certain level of authentication throughput can be guaranteed by stopping photographing. The photographing is continuously retried if these stop conditions are not satisfied, but when the stop conditions are satisfied, the failure of authentication is determined.

In the processing flow described above, an example of processing for accurately acquiring the structure of the blood vessel image from the transmitted image obtained by the three-wavelength light source will be described in detail. In the description below, wrinkles, blurs, etc. may generally be referred to as noise.

It is commonly known that an absorption coefficient or a scattering coefficient of light in blood vessels or skins changes according to the wavelengths. In this example, therefore, transmitted images of three different wavelengths are photographed in a time-dividing manner, and, by using these three images, wrinkle information of the finger skin that have been projected in the images is removed, and blurs of the blood vessel image is are reduced. Herein, it is assumed that wavelength $\lambda_0$ has a high absorption ratio in blood, wavelength $\lambda_2$ has an absorption ratio in blood equivalent to that of the wavelength $\lambda_0$, and wavelength $\lambda_1$ has an absorption ratio in blood lower than the former two wavelengths. In addition, a diffusion coefficient over the skin of the wavelength $\lambda_0$ is twice as large as that of the wavelength $\lambda_2$. In this example, the wavelengths satisfying the above conditions are set as the wavelength $\lambda_0$ of 660nm, the wavelength $\lambda_1$ of 690nm, and the wavelength $\lambda_2$ of 870nm, respectively. Other combinations of wavelengths are also possible so long as the above conditions are satisfied.

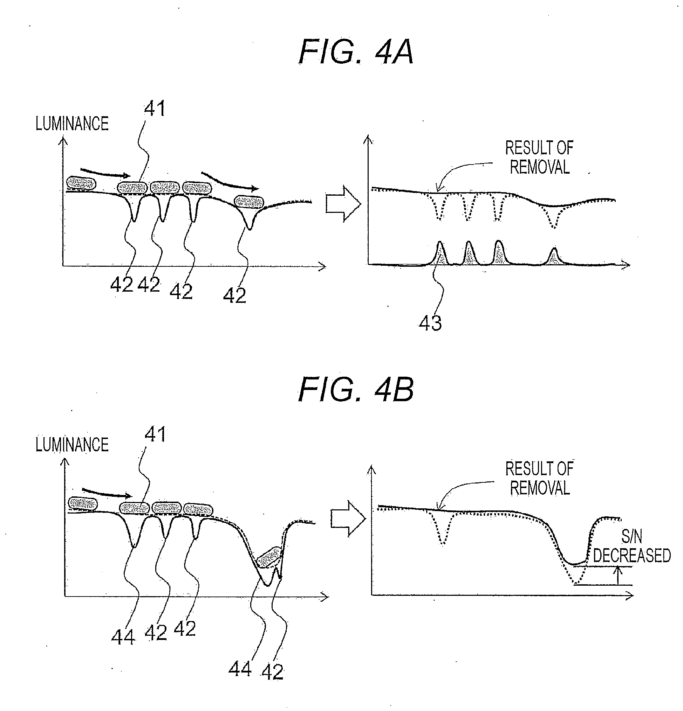

Prior to the description of this example, an outline of a common wrinkle removing technique of the past will be described by referring to FIGS. 4A and 4B. In the past technique, a basic method is to separate wrinkles from the blood vessel in an observed image according to features of the image that have been photographed by a single wavelength. To remove high frequency components, a method of filtering a specific spatial frequency, top-hat conversion, or a method of analyzing independent components may be used. In this example, the most representative method of top-hat conversion is used to remove the high frequency components. Specifically, a function called a structuring element 41 is defined corresponding to the noise component desired to be removed from the luminance curve of a cross-section of the image. Then a difference between the trajectory of sliding this function on this curve and the original curve is taken as a wrinkle component 43. This process is illustrated in FIG. 4A. In this example, all recesses in the lower part are regarded as wrinkle shadows 42. When the wrinkle components 43 are detected and reduced from the original signal components, the shadow of the wrinkle pattern is removed.

To adjust the luminance components that are desired to be removed as noise, only the length of the structuring element 41 can be adjusted. Since the wrinkles in the skin are often narrower than the width of a blood vessel, this adjustment has been performed empirically in an attempt to remove the wrinkle components within the largest possible range in the past method. As illustrated in FIG. 4B, however, the image often includes not only the wrinkle shadow 42 but also shadow 44 of the blood vessel, which makes it difficult to discriminate the blood vessel shadow 44 from the wrinkle shadow 42 of the skin according to the width and brightness of the shadow. In this case, applying the past method may lead to erroneous removal of the blood vessel shadow 44, as illustrated in FIG. 4B and, additionally, may also cause a problem of decreasing the contrast (S/N) of the shadow 44 of a clearly imaged blood vessel. This may cause a problem that the finger vein pattern that is effective in personal discrimination may include deficiency and authentication accuracy may be degraded.

In this example, therefore, a method of removing epidermis image components that have been imposed on the blood vessel image is applied as a method for accurately photographing the blood vessel images using a two-wavelength light source. It is assumed in this example that the wrinkles of the wavelength $\lambda_0$ are removed by two wavelengths $\lambda_0$, $\lambda_1$, and the wrinkles of the wavelength $\lambda_2$ are removed by two wavelengths $\lambda_1$, $\lambda_2$.

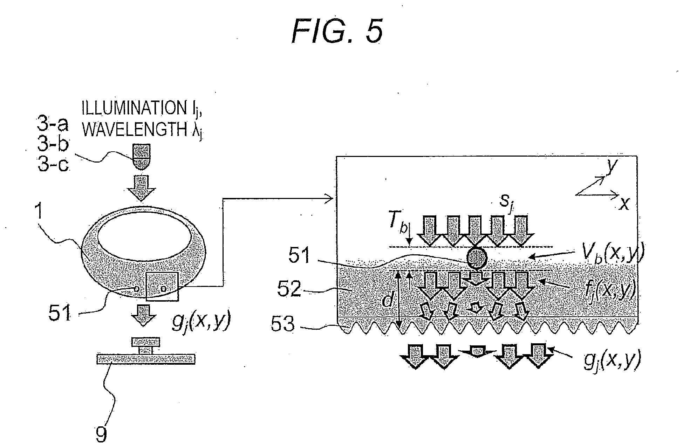

FIG. 5 is an exemplary conceptual view illustrating an optical behavior model of the inner part of a living body. Illuminated light $I_j$ of a wavelength $\lambda_j$ is incident on the back of the finger and proceeds downwards to the lower part of the drawing, while scattering in a living body, to reach above a blood vessel 51 (finger vein near the palm side) which are distributed near the skin on the palm side. The blood vessel 51 is buried in a layer formed by dermis 52, and this layer is regarded as a blood vessel layer. It is assumed that the living body is a strongly scattering body and light distribution $s_j$ over the blood vessel layer is uniformly distributed. The light distribution $s_j$ attenuates by hemoglobin in blood contained in the blood vessel and reaches under the blood vessel to form a light distribution $f_j$. The light distribution $f_j$ is formed by attenuation of the internal light $s_j$ in blood according to Lambert-Beer law, and is written as

$$f_j = s_j \exp\{-\mu_{bj}V_bT_b - \mu_{dj}(1 - V_b)T_b\} \ldots\ldots\ldots (1)$$

where $\mu_{bj}$, $\mu_{dj}$ represent coefficient of extinction (a sum of coefficient of absorption and scattering) in blood and skin at the wavelength $\lambda_j$, $T_b$ represents a thickness of the blood vessel layer, $V_b$ represents a volume ratio of the blood vessel occupation in the dermis in the range of a thickness $T_b$ and satisfies $0 \leq V_b \leq 1$. Two-dimensional images are used, and the description of coordinates thereof will be omitted for the sake of easiness of understanding by simply writing $f_j (x, y)$ as $f_j$. In addition, it is assumed that the optical scattering in the blood vessel section is not considered. The coefficient of extinction may be defined by either the coefficient of absorption or the coefficient of scattering.

In this state, $f_j$ represents the image itself of the blood vessel in the body, and can be regarded as a high definition blood vessel image, as it does not include any influence of blurs caused by optical scattering in tissues of the living body or attenuation on the surface of the skin, etc. An object of accurate measurement of the blood vessel image is, therefore, to acquire the blood vessel image $f_j$ in the body. The light distribution $f_j$, however, cannot be observed directly, because $f_j$ proceeds by scattering in subcutaneous tissues (dermis and epidermis) on the palm side, or receiving influence of the optical attenuation $c_j$ on the surface of the skin, such as the wrinkles 53 of the skin, before photographed by the camera. In particular, it is more difficult to estimate $f_j$ in the body, as the optical scattering in the body changes depending on the depth position of the blood vessels. By receiving such a change in luminance $f_j$ is dissipated from the surface of the finger. A light distribution here is regarded as $g_j$. Since $g_j$ can eventually be photographed by the camera, $g_j$ is referred to as an observation image.

In view of the above behavior, the observation image $g_j$ is formulated as

$$g_j = (f_j * h_j)c_j + n_j \ldots\ldots\ldots (2)$$

where $h_j$ represents a point spread function (PSF) which indicates the degree of light diffusion in the subcutaneous tissue (dermis and epidermis), $n_j$ represents a noise component of the image, and an operator "*" represents a convolution operation. Because of scattering in the subcutaneous tissue, the true blood vessel image $f_j$ would be attenuated in the observation image $g_j$ and acquired in a more blurred manner. The blurs in the blood vessel image due to the light diffusion might have an influence in terms of accurately photographing the blood vessel image, but it is regarded herein that the influence of light diffusion is sufficiently smaller than that of the epidermis image. The object herein is to remove the epidermis image, and the model would be modified into the one that can be analyzed, more easily. Specifically, instead of expressing the diffusion by the convolution operation of the PSF, the model would be written by considering the attenuation of light alone according to Lambert-Beer law. At this time, formula (2) is written as the below.

$$g_j = (f_j \exp\{- \mu_{dj}d\})c_j + n_j \approx s_j \exp\{- \mu_{bj}V_bT_b - \mu_{dj}(1 - V_b)T_b - \mu_{dj}d - \mu_{cj}V_cT_c\} \dots\dots (3)$$

where d represents a depth of the blood vessel (a thickness of the skin), $\mu_{cj}$ represents coefficient of extinction in the wrinkles of the skin, $V_c$ is a volume ratio of the wrinkles of the skin occupying the epidermis, and $T_c$ is a thickness of the wrinkle layer of the skin. From formula (3), it is known that that the observation image $g_j$ has been formed as a result of attenuation of the light distribution $s_j$ in the living body according to an amount of blood, the thickness of the skin, and wrinkles of the skin.

By the formulation above, the issue of sharpening the blood vessel image, which is an object of this example, comes to an issue of acquiring $f_j$ of formula (3) from the observation image $g_j$.

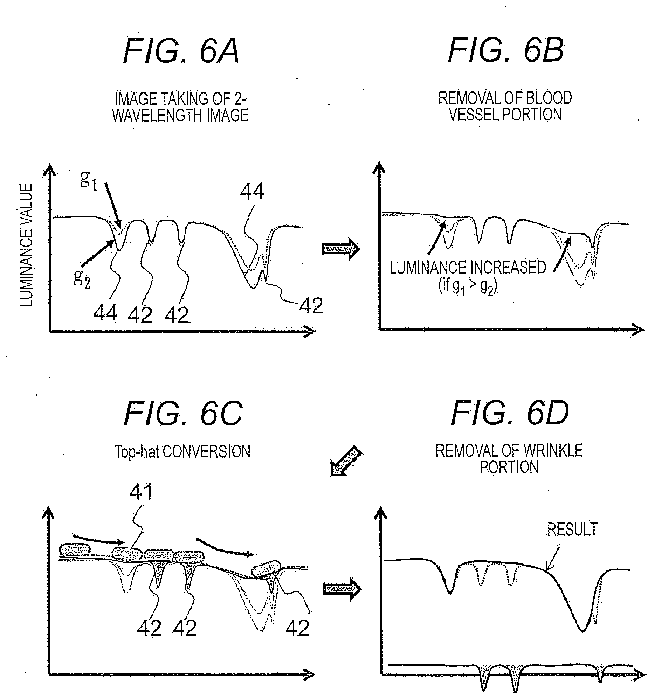

FIGS. 6A to 6D illustrate processing procedures for removing wrinkles of the skin. First, as illustrated in FIG. 6A, images of two different wavelengths having different coefficient of extinction in the blood are taken continuously. The light of two different wavelengths may be provided using different light sources for each wavelength, or a single light source may be used to emit such light of two different wavelengths. Although an example of removing wrinkles related to $g_2$ is illustrated in the following description, the removal of wrinkles of $g_0$ can also be possible by replacing $g_2$ by $g_0$, and, regarding a subscript j that represents a wavelength, such as $g_j$ and $\lambda_j$, replacing j = 2 by j = 0.

$g_1$ represents an image of the wavelength $\lambda_1$ having a low coefficient of extinction in the blood, and $g_2$ represents an image of the wavelength $\lambda_2$ having a high coefficient of extinction in the blood. Looking at a portion 44 of the blood vessel, it can be seen that the luminance value satisfies $g_1 > g_2$, but at a wrinkle portion 42 of the skin, the luminance values are nearly identical, or $g_1 \leq g_2$. According to such a difference, the blood vessel region is separated from the noise region that has been formed by wrinkles, foreign objects, etc. Although it seems possible to simply acquire the information of the blood vessel portion .by determining the difference image between $g_1$ and $g_2$, the difference is not sufficient for completely removing the information of wrinkles alone, as the luminance value of the wrinkles of the skin are slightly different. Therefore, completely processing the information of wrinkles, that is, the difference in the noise information would significantly contribute to the authentication accuracy.

Next, as illustrated in FIG. 6B, the shadow of luminance caused by the blood is removed from $g_2$. By taking the natural logarithm is taken on both terms of formula (3) above, a resulting formula is as the below.

$$\ln g_j \approx \ln s_j - \mu_{bj}V_bT_b - \mu_{dj}(1 - V_b)T_b - \mu_{dj}d - \mu_{cj}V_cT_c \dots (4)$$

In this state, by multiplying $g_2$ by a certain coefficient such that the luminance value of both images is identical for pixels in which the volume ratio $v_b$ of the blood becomes zero, then a result is as the below.

$$\ln s_2 - \mu_{d2}T_b - \mu_{d2}d - \mu_{c2}V_cT_c = \ln s_1 - \mu_{d1}T_b - \mu_{d1}d - \mu_{c1}V_cT_c \dots (5)$$

From two formulae of formula (4) where j = 1, 2, and three simultaneous equations of formula (5), an unknown number $V_bT_b$ is obtained. When the variable $V_bT_b$ is defined as a blood light shading coefficient $V_{bt}$, then $V_{bt}$ is written as the below.

$$V_{bt} = (\ln g_1 - \ln g_2)/(\mu_{b2} - \mu_{b1} + \mu_{d1} - \mu_{d2}) \dots (6)$$

When the luminance value of a region satisfying $V_b > 0$ is the same as the luminance value of a region satisfying $V_b = 0$, the shadow caused by the blood can be removed from the observation image $g_2$. Specifically, coefficient $\beta$ satisfying $\beta g_j = g_j^{(Vb = 0)} \dots (7)$ should be determined, where $g_j^{(Vb = 0)}$ represents a luminance value at $V_b = 0$ in the vicinity of a pixel (x, y) in $g_j(x, y)$. Formula (7) is modified to obtain formula (8) below, to which formula (4) and the expression given when $V_b = 0$ are assigned in the formula (4) to obtain formula (9) below.

$$\ln \beta = \ln\{g_j^{(Vb=0)}\} - \ln\{g_j\} \ \ldots\ldots \ (8)$$

$$\therefore \beta = \exp\{\mu_{b2} - \mu_{d2}\}V_{bt} \ \ldots\ldots \ (9)$$

Summarized, the image $g_{2e}$ that has removed the shadow caused by the blood vessel can be acquired from the observation image $g_2$ as the below.

$$g_{2e} = g_2 \exp\{\mu_{b2} - \mu_{d2}\}V_{bt}, (\text{if } V_b > 0) \ \ldots\ldots \ (10)$$

$$= g_2, \qquad (\text{otherwise}) \ \ldots\ldots \ (11)$$

The result of this processing is illustrated in FIG. 6B.

In the image $g_{2e}$ where the shadow of the blood vessel has been removed from the image $g_2$, only the noise information other than the information of the blood vessel, such as the wrinkles of the skin, is left. A common top-hat conversion is performed on such an image including the noise region to extract high frequency components, such as the wrinkles of the skin. This process is illustrated in FIG. 6C. Assuming that a noise image $c_h$ only includes the extracted wrinkle components and an operator for the top-hat conversion is TH($\cdot$), then the image $c_h$ formed by the wrinkle components is written as the below.

$$c_h = TH(g_{2e}) \ \ldots\ldots \ (12)$$

Although the wrinkle components have been removed in this example by using the top-hat conversion, as it can be implemented relatively easily and operate at a high speed, a common noise removing method represented by methods such as independent component analysis, graph-cut, and filtering of a specific frequency of the image, can also be used.

Lastly, from the image $c_h$ including the wrinkle components alone and the original image $g_2$, an accurate blood vessel image $g'_2$ in which the wrinkle components alone have been removed is acquired by the below.

$$g'_2 = g_2 - c_h \exp\{-(\mu_{b2} - \mu_{d2})V_{bt}\} \ \ldots\ldots \ (13)$$

This process is illustrated in FIG. 6D. Thus, the shadow caused by the blood may be left, but still the wrinkle components of the skin, which are deemed unnecessary for authentication, can be selectively removed, to thereby obtain the blood vessel image for authentication including a true blood vessel image. Accordingly, the finger vein authentication robust to changes of the presence or absence of the wrinkles of the skin can be realized.

This technique is applicable not only to the removal of the wrinkles and fingerprints on the surface of the skin of fingers, but also to the removal of skin stains or folding wrinkles of semi-transparent gloves such as sanitary gloves. Accordingly, the finger vein authentication that is robust to finger stains and can authenticate fingers even when the semi-transparent gloves are put on can be realized.

If the removed noise information is wrinkle information unique to the skins, such as fingerprints, the information can also be used as new authentication information. Since the information of the veins and fingerprints can be acquired at a time by a single optical system, multimodal authentication can be realized without additionally providing an apparatus for taking fingerprint images.

Next, an example of removing blurs of the blood vessel image of the image $g_0$ in which the acquired wrinkles have been removed, and the image $g_2$ will be described.

Generally, it is known that, of two wavelengths having a relation that one wavelength has an optical extinction coefficient in the skin twice as large as that of the other wavelength, the light of the wavelength having the lower coefficient of extinction expands less than the light of the other wavelength. In addition, if a point light source virtually exists in the skin, the light expansion is, when observed from the outside, wider when such a point light source is located deeper in the skin. When the light expansion, which is observed from the outside of the skin, of the above two wavelengths is compared with each other, it is known that the light expansion is nearly the same when the point light source of the wavelength having a smaller coefficient of extinction is located at a position twice as deep as that of the point light source of the other wavelength.

Using such a characteristic, it is possible to estimate the degree of expansion of light in the blood vessel image when the blood vessel images $g_0$ and $g_2$, which have been photographed by two different wavelengths, have the relation of doubled coefficient of extinction of light in the skin. Specifically, the image from which the wrinkles of the skin has been removed is formulated as $g_j = f_j*h_j + n_j$ ....... (14). When $j = 0$ and $j = 2$, formula $h_0 = h_2*h_2$ ....... (15) is satisfied correspondingly. By solving the formula on the true blood vessel image f, $f = g_2 + (g_0 - g_2)*g_2*g_0^{-1}$ ....... (16) where $a*b^{-1}$ represents a deconvolution operation of the image a by the image b.

It is possible to acquire the blood vessel image f that includes no blurs by calculating the above formula. The deconvolution operation may be performed by a common method using a Weiner filter, constrained least square filter (CLS), total variation (TV), bilateral total variation (BTV), etc.

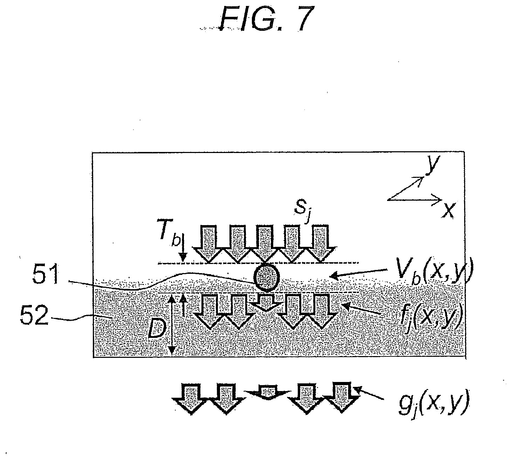

FIG. 7 is an exemplary model of the inner side of the living body to measure a depth of the blood vessel from a two-wavelength transmission image. Acquisition of the depth of the blood vessel is important in that, as in the removal of wrinkles or blurs on the surface of the skin described above, information of the true structure of the blood vessel is acquired to increase the information amount that can be used in authentication. The model of FIG. 7 is based on situations substantially similar to those of FIG. 5, except that, in FIG. 7, the model has been simplified on the premise that no wrinkles are present in the skin and no light diffusion occurs in the dermis layer 52. In practice, such a simplified model is applied to the measurement of the depth of the blood vessel by using a two-wavelength image after the wrinkle removal processing has been performed.

In a physical model illustrated in FIG. 7, if a blood vessel concentration $V_{bt}$ is solved, a depth D at which the blood vessel exists can also be determined. When the internal light $s_i$ attenuates according to the Lambert-Beer law, the observation image $g_i$ is written as $\ln(g_i) = \ln(s_i) - \mu_{bi} V_b T_b - \mu_{si}(1 - V_b)T_b - \mu_{si}D$ ....... (17) where $\mu_{bi}$, $\mu_{si}$ represent coefficient of extinction of the blood vessel and the skin, respectively. Images are photographed by two wavelengths to acquire $g_1$ and $g_2$, which are multiplied by coefficient $\alpha_i$ at a position where no blood vessel exists (i.e., $V_b = 0$) to satisfy $\alpha_i g_1 = \alpha_2 g_2$ (= constant), and the formula becomes $\ln(\alpha_1 s_1) - \mu_{s1} (T_b + D) = \ln (\alpha_2 s_2) - \mu_{s2}(T_b + D)$ ....... (18). From i = {1, 2} of formula (17) and formula (18), $V_b T_b(= V_{bt})$ can be determined by $V_{bt} = (\ln(\alpha_1 g_1) - \ln(\alpha_2 g_2)) \div (\mu_{b2} - \mu_{b1} + \mu_{s1} - \mu_{s2})$ ....... (19). Finally, formula (19) is assigned to formula (17) (where i = 2), then the depth D of the blood vessel can be derived as

$$D = (\ln(\alpha_2 s_2) - \mu_{s2}T_b - \ln(\alpha_2 g_2) + (\mu_{s2} - \mu_{b2})V_{bt}) \div \mu_{s2} \cdots (20)$$

where D is not fixed, as the internal light $s_2$ and the thickness $T_{bis}$ of the blood vessel layer cannot be observed. Meanwhile, as $g_2$ is multiplied by coefficient $\alpha_2$, the term of the internal light $\ln(\alpha_2 s_2)$ can be regarded as a fixed value in any place. In addition, if $T_b$ is always constant for any finger, $\ln(\alpha_2 s_2) - \mu_{s2}T_b(= C)$ can be at a fixed value. At this time D is defined as a relative depth $D_r$ of the blood vessel. Specifically, a relative depth of the blood vessel having a biased fixed value C can be acquired. It is noted, however, $C > \ln(\alpha_2 g_2)$ according to the physical constraint that the light attenuates. As described above, the amount of information useful for the personal authentication increases compared to the past method that uses only a planar blood vessel structure. Thus, improvement of authentication accuracy can be expected.

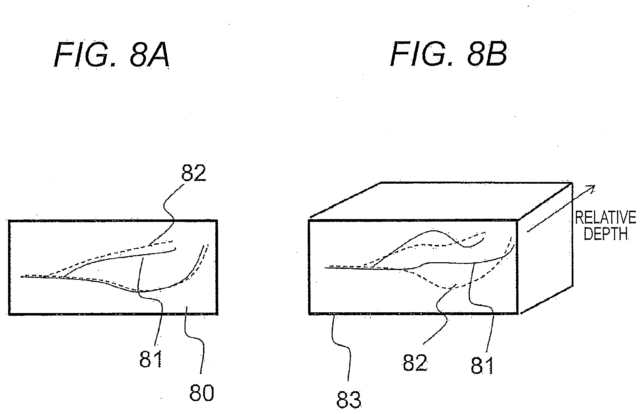

FIGS. 8A and 8B are schematic views illustrating an example where different blood vessel patterns can be discriminated properly by using the depth of the blood vessel. FIG. 8A illustrates two different finger blood vessel patterns 81 and 82 overlapped with each other to provide a conventional two dimensional projection image of the blood vessel 80. Since the two dimensional projection images of both patterns have originally been similar to each other, it can easily be determined by the past method that these patterns are in the same pattern. Meanwhile, when such images are overlapped to provide a three-dimensional blood vessel image 83, as illustrated in FIG. 8B, it is observed that the both blood vessel patterns 81 and 82 have a large difference in shape in the depth of the blood vessel. Accordingly, it would be determined more often that the both images are of different fingers. This contributes to the improvement of authentication accuracy compared to the authentication method of the past using the two-dimensional projection images.

To collate three-dimensional line patterns, a common collating technique of three-dimensional shapes, including the methods such as three dimensional template matching (voxel matching), a method of determining the degree of similarity between feature points by measuring the SIFT feature amount, vector collating to make vectors of tangential line components of a line and calculate an inner product thereof may be used.

An example of collation using a three value template - and a relative depth distribution will be described. In this example, a collating method applies the above depth information to the three value template.

First, a three-value template Pv of finger veins is extracted. PV is an image obtained by extracting features from the blood vessel image and classifying the extracted features into three values corresponding to blood vessel/ambiguity/background according to the likeliness of the blood vessel of the features. Next, a relative depth distribution Dr is obtained, as mentioned above. Lastly, the value of Dr is normalized to 0 to 255 to provide the value D'r. Examples of normalization include a method of determining an average value da and a dispersion $\sigma$ of Dr at all positions of pixels of the blood vessel, and linearly converting values within the range da$\pm 3\sigma$ to 0 to 255. Final collating data of the presented method will be a pair of Pv and D'r.

Next, an example of the collating method using the relative depth distribution mentioned above will be described. The example is based on a method that the number of overlapping portions (mismatches) between the blood vessel and the background, which have been obtained by overlapping a registration Pv and an input Pv, is divided by a total number of pixels of the blood vessel to obtain a mismatch ratio. In contrast, this example determines a three-dimensional gap as a mismatch, even when the blood vessels are overlapped each other, so long as a difference between the relative depths D'r of the blood vessels assigned to those pixels exceeds a threshold value ThH. Thus, the problem of the past method, that is, the increase of similarity due to coincidental overlapping of the pixels of the blood vessels of different fingers, can be alleviated. Further, it is determined for pixels having the difference of D'r smaller than ThL, due to overlapping of the blood vessels, that the similarity is higher than usual. Then the number of pixels is weighted by multiplication, and a result is reduced from the number of mismatches to decrease the mismatch ratio. Accordingly, the fingers that are similar in both the shape of the blood vessel and the depth can further be separated from the different finger distribution.

Specifically, the mismatch ratio between the three value template images to which the depth of the blood vessel are assigned are expressed as the below.

```
Mismatch ratio = {α₁ × number of overlap of blood
vessel and background + α₂ × number of overlap of blood
vessels having depth difference larger than ThH - α₃ ×
number of overlap of blood vessels having depth difference
smaller than ThL}/total number of pixels of blood
vessel ....... (21)
```

where $\alpha_i$ (i = 1, 2, 3) represents a weight for each mismatch number. For example, the wavelengths may be $\lambda_0$ = 690 [nm], $\lambda_1$ = 870 [nm], $\alpha_1 = \alpha_2 = \alpha_3$ = 1, ThL = 16, and ThH = 80. In particular, the wavelengths may be selected, as in this example, such that the wavelengths have a large difference of light absorption level in the blood, to thereby allow stable extraction of information of the depth of the blood vessel.

In the above method, in calculating the mismatch ratio, the calculation of the mismatch ratio is combined by the depth information in the three-value template with the weight of $\alpha_i$ assigned thereto. Alternatively, it may also be possible to calculate a collating score in advance according to the past technique, followed by determining the similarity level of the depth maps by a method such as' normalization mutual correlation and combining the collating score of the past technique with the similarity level regarding the depth map. In this case, the final collating score can be obtained as a multidimensional vector. In contrast, a determination method for optimally discriminating a person from others may be obtained by a statistical method. For example, parameters that can best discriminate the person from others are obtained from a learning sample according to discrimination analysis or main component analysis, and scores are combined linearly to degenerate to a single collating score. An effect of improving authentication accuracy can be expected by using such a statistical method. Otherwise, it may also be possible to perform authentication by determining a border with which the person can be discriminated from others according to a support vector machine (SVM).

Meanwhile, the light source of the authentication apparatus according to this example may correspond to two wavelengths or a single wavelength, by considering necessary authentication accuracy, functions, cost, etc. As described above, when the light source corresponds to three wavelengths, the removal of wrinkles of the finger, the removal of blurs of the blood vessel image, and the estimation of the depth of the blood vessel are allowed. It is also possible to restrict each function and the wavelength may be set to include the necessary wavelength. By limiting the number of types of wavelengths, such effects as high speed imaging, improvement of endurance to outside light, lower cost of the apparatus, simplification of the control circuit, etc. can be obtained. For example, the wavelengths can be limited to the above $\lambda_1$ and $\lambda_2$ to realize the removal of wrinkles of the skin and the measurement of the depth of the blood vessel. Alternatively, the wavelengths can be limited to the above $\lambda_0$ and $\lambda_2$ to realize only the removal of blurs of the blood vessel. In particular, regarding the removal of blurs, enhancement processing, such as unsharp masking, deconvolution of a PSF that assumes light diffusion in the body, or blind deconvolution that estimates light diffusion in the body to remove blurs, can be applied to realize simplified enhancement of the blood vessel images. Therefore, it would be highly practical to use a combination of a method of removing blurs without using the optical features and a method using two wavelengths $\lambda_1$ and $\lambda_2$.

A particular example of removing blurs of the blood vessel based on a single image is a method according to the maximum a posterior (MAP) estimation based on Bayes' theorem. The observation result of the luminance information of the blood vessel portion is provided as a result after the true blood vessel portion existing in the body has been blurred or superimposed with noise due to the influence of diffusion of light or the like. It is assumed that the true blood vessel does not include noise and keeps connectivity, and no steep change is present in the spatial luminance anywhere other than at edge portions of the blood vessel. An estimation result of the true blood vessel matching such a condition is generated in advance and, after blurring the estimation result by a function formulating the light diffusion, is compared with the observation image. If the estimation result is similar to the true blood vessel, the blurred image is supposed to be similar to the observation image. To estimate the blood vessel image, therefore, the estimation result of the true blood vessel is corrected so as to minimize the error between the observation image and the blurred image of the estimated value, and this process is repeatedly performed to estimate the blood vessel image.

Alternatively, for a database of a large volume of finger images, samples such as a collection of samples of blood vessel portions alone, a collection of samples of skin wrinkles alone, or a collection of samples in which no blood vessels or wrinkles are included are prepared, and the feature of luminance such as a luminance curve in a cross-section, is learned in advance. When an unknown image is given, it is determined to which class the given image most likely belongs according to a partial image thereof, and it is also determined whether it is stochastically of the blood vessel portion. Such a method is also effective. By such processing, the blood vessel portion alone can be enhanced even in a single image. Similar processing can also be applied, indeed, to an image having multiple wavelengths.

The blood vessel image $g_2$ taken by using the wavelength $\lambda_2$, as defined in this example, is an image preferable for acquiring an amount of features to be used in authentication, because clear blood vessel patterns are included compared to the images using other wavelengths. In limiting the wavelengths to be used according to necessary functions and costs, the removal of wrinkles or blurs can be performed especially for $g_2$ which is then used for authentication to improve the authentication accuracy.

In the above example, since $g_1$ is the observed image using the wavelength with which the blood vessel is hardly seen, the removal of wrinkles is not performed for $g_1$ itself which, instead, is only used as a reference image for removing wrinkles of other images. By modifying the above equations, however, the wrinkles in $g_1$ can also be removed. There may be a case where a clear blood vessel is observed even in the image $g_1$ depending on the selection of wavelength, and it is possible, in this case, to remove wrinkles in $g_1$ to convert it to a clear blood vessel image which may subsequently be used in authentication processing or the like.

Next, an example of collating processing using a correlation between the pressure applied to a finger and biometric information to be obtained will be described in detail. First, a relation between pressure and time is described by illustrating a typical example of transition of pressure values between the start and stop of photographing, followed by a description of control of acquiring variations of pressure values. After that, a relation between the amount of change of the pressure and the amount of change of patterns will be described. Lastly, collating processing that considers the amount of change of the pressure and the amount of change of patterns will be described.

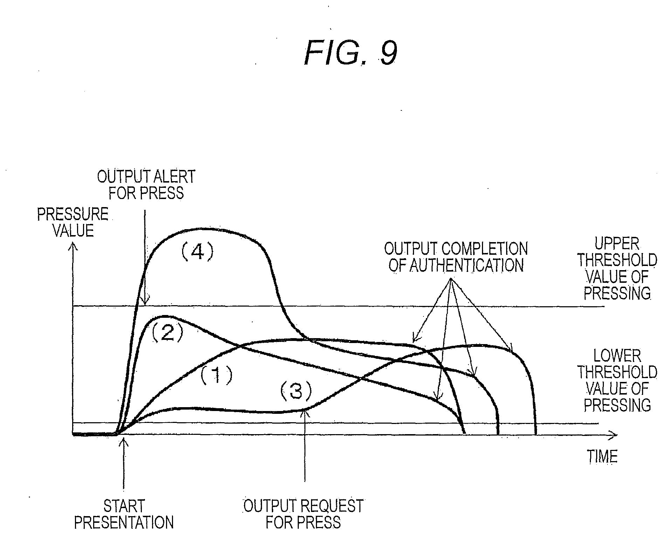

FIG. 9 is an explanatory view illustrating an example of a relation between a change of pressure with time and timing of ending the photographing.

In this embodiment according to the invention, four pressure sensors are provided, and a total output of the four pressure sensors is regarded as an intensity of pressure (pressure value) applied by a finger. Most typically, the transition of the pressure value after the start of image taking is, as illustrated by a curve (1) of FIG. 9, such that a pressure value increases gradually as time passes from the state in which the finger is put in a stationary manner, and finally converges to a fixed value. Alternatively, as illustrated by a curve (2), the apparatus may be pressed relatively strongly by a fingertip at the start, with the pressure gradually decreasing but not converging to a fixed value. As illustrated by a curve (3), the application of an extremely weak pressure may be continued. There may be a case where, as illustrated by a curve (4), a pressure value that is largely exceeding an expected pressure value is applied upon presentation of the finger. In such a case, the pressure increases steeply from zero. In another case, the pressure may return to zero steeply even when the pressure that has been applied is very strong.

To correspond to these variations of applying the pressure, the following conditions are set for taking an image in this embodiment. First, upper and lower limits of the pressure value of a finger that is suitable for photographing are defined. In observing the pressure value in time series, if the pressure value exceeds the upper limit, "output of a pressing alert" is performed to provide feedback to a user that the pressing is too strong, and recommend the user to weaken the pressure. Similarly, if the pressure value is below the lower limit, "output of a pressing request" is performed to recommend the user to press the finger on a presentation part. The lower value may be set to nearly zero, as the blood vessel of the finger can often be observed in practice even when the pressure is weak.

Further, as illustrated by the curve (3), a state where the pressure value exceeds the lower limit but is still weak is continued, and an image of a stronger pressure has not been taken, the "output of a request for press" is performed to recommend the user to lightly press the apparatus to exert pressure. Similarly, there is a case where the pressure value increases before an image of a weak pressure value has been taken, due to a reason associated with a frame rate of the camera, reaches to nearly the upper threshold value, and continues to be at a constant value. In such a case, as the weak pressure image has not been taken yet, guidance is presented to decrease the pressure of the finger. By presenting such guidance, images of various pressure values in a range between the upper and lower limit values can be obtained. Since the amount of information to be used in authentication increases, the authentication accuracy can be improved.

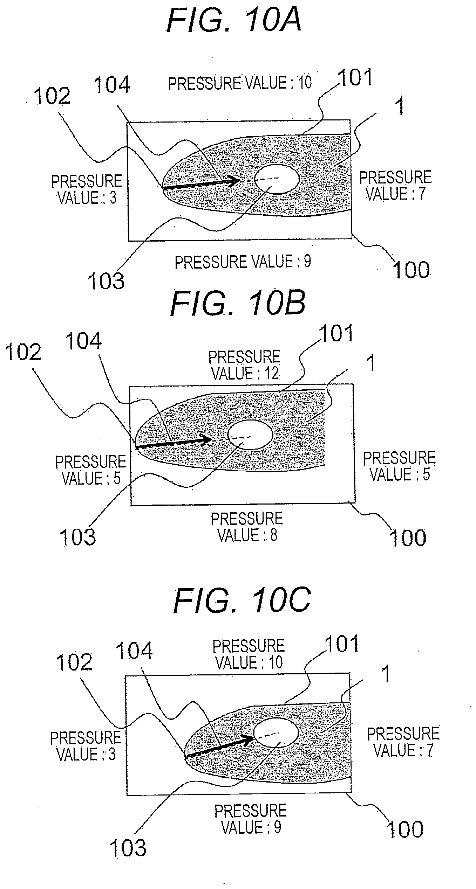

FIGS. 10A, 10B and 10C are explanatory views illustrating the concept and a calculating method of pressure vectors.

FIG. 10A illustrates exemplary pressure values of four pressure sensors corresponding to the presentation of a finger. FIG. 10B illustrates an example in which the same finger as in FIG. 10A has been placed with the same pressure at a different location. The finger 1 is projected on a finger image 100, and a finger contour 101 is detected. A fingertip is located on the left side of the image, and a tip of the finger contour 101 is regarded as a fingertip 102. In addition, an oval-shaped pressed region 103 that is generated when the pressure value between the finger and the acrylic plate contacting the finger is at or more than a predetermined value. On the four sides of the finger image 100, a pressure value of each pressure sensor is indicated.

From the comparison of these two views, it can be seen that the finger 1 is presented at different positions, but relative positions within the finger contour where the pressed region 103 is observed are the same. Thus, even when the entirely the same pressure has been applied, as in this embodiment, a balance of the four pressure values obtained are different due to displaced positions of the finger when it contacts the acrylic plate. Although the same pressure has essentially been applied for presentation, it cannot be regarded as such by simply comparing the four pressure values. In this embodiment, therefore, the finger contour 101 is used to define a pressure vector capable of normalizing the location where the pressure has been applied.

Next, an exemplary method for calculating a pressure vector will be described. First, the finger contour 101 is detected as described above, and the fingertip 102, which is a portion with high curvature in the contour line located on the left side of the drawing, is regarded as a reference point. A pressure vector 104 is defined as having a direction towards the center of the pressed region 103 from the fingertip 102. The reference point of the pressed region may be another predetermined reference point, other than the one in the direction towards the center. The center position of the pressed region 103 can be determined according to a ratio of the pressure values of the four pressure sensors. Specifically, when the gravity of arrangement of four pressure sensors matches the center point of the four pressure sensors, the center of the pressures is determined according to a pressure value ratio for a pair of two pressure sensors located symmetrically relative to the center point of the finger image. For example, in FIG. 10A, it is considered that the center of the pressure is at a 7/10 position from the left end towards the right across the width of the image, as the values of the right and left pressure sensors of the drawing are 3 and 7, respectively. Similarly, the center of the pressure in a vertical direction is considered to be at a 10/19 position of the height of the image from the top end towards the bottom. The magnitude of the pressure vector is regarded as matching the pressure value mentioned above, and can simply be represented by a total of four pressure values.

When the pressure vectors 104 of FIGS. 10A and 10B are compared, it is known that both pressure vectors 104 almost match with each other.

As mentioned above, when the apparatus is small and no positioning means is provided in the housing, a large variation or positional deviation may occur at a place where the user puts a finger on every time the image of the finger is taken. On the other hand, even though the position of the finger is different every time the image is taken, it is known statistically that a relative position of the pressed region in the finger area, which is generated when the finger is placed, would be substantially the same due to personal habits or the like. The pressure vector according to the embodiment of the present invention is information generated by using such a characteristic.

Although the pressure vector from the reference point of the finger to the center of the pressed region has been used above, it may not be possible to use the finger contour to determine the similarity, and the positional relation of the pressed region of each image may be used instead.

FIG. 10C illustrates an example where the four pressure values completely match those of FIG. 10A, but the finger position is different. When the pressure vector is determined to compare these examples, it can be properly determined that the pressure is applied differently.

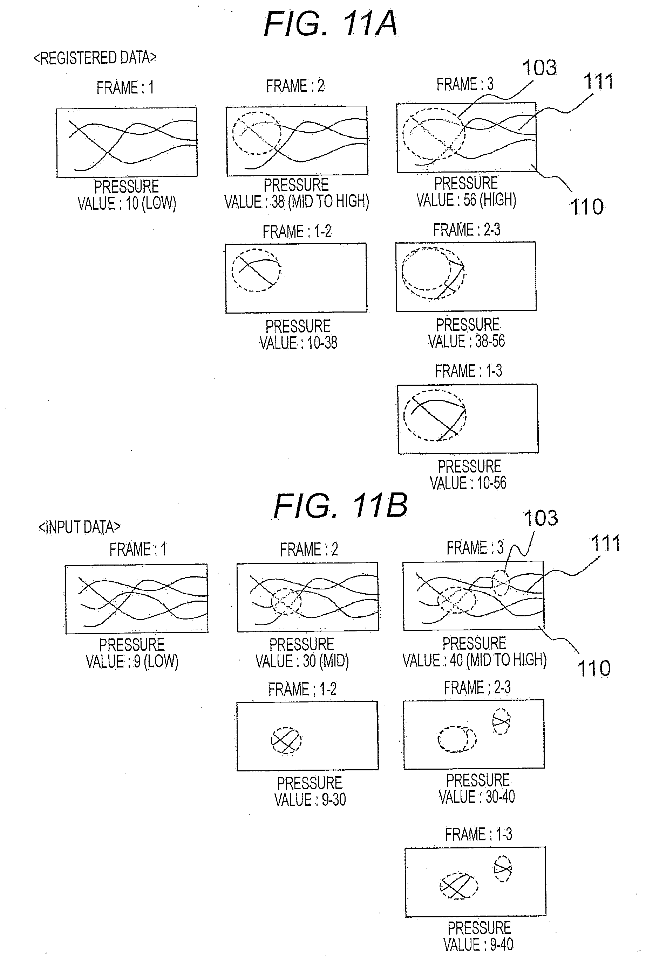

FIGS. 11A and 11B are explanatory views illustrating an example of collating data using the measured pressure

FIG. 11A is exemplary registered data in which registered collation data 110 and pressure values are arranged in time series. Frames are photographed in order from frame 1, and frame 3 is the last photographing state. Blood vessel patterns 111 are distributed in the collation data 110. The pressed region 103 is indicated by a dotted line, and the blood vessel image within this region has been erased. A pressure vector is also registered for each frame. Herein, the pressure values alone are indicated, since the directions of the pressure vectors are regarded as nearly the same. In this description, the pressed region in the registration data may sometimes be referred to as a registered pressed region. In addition, the pressure values and the pressure vectors that have been stored as the registered data may also sometimes be referred to as registered pressure values and registered pressure vectors, respectively.

In this example, the pressure value gradually increases as the frames are photographed successively, from which it is known that the pressure is gently applied. As the pressure value increases, it can be seen that the pressed region 103 expands correspondingly. For each frame, a difference image of a deficiency area of the pattern is also registered. For example, the collation data referred to as "Frame: 1 - 3" in the drawing represents a difference image between frames 1 and 3. The difference images may be stored in a database in advance, or alternatively generated during collation. This can be designed freely by considering a tradeoff between the capacity of the database and processing time to be taken.

Similarly, FIG. 11B is exemplary input data, in which the blood vessel patterns and pressure values are arranged in time series. When the input finger is presented, the finger vein pattern and the pressure vector are obtained by the processing described above. In this example, it is assumed that three finger vein patterns have been input, and the direction component of the pressure vector is similar to that of the registered pressure vector.

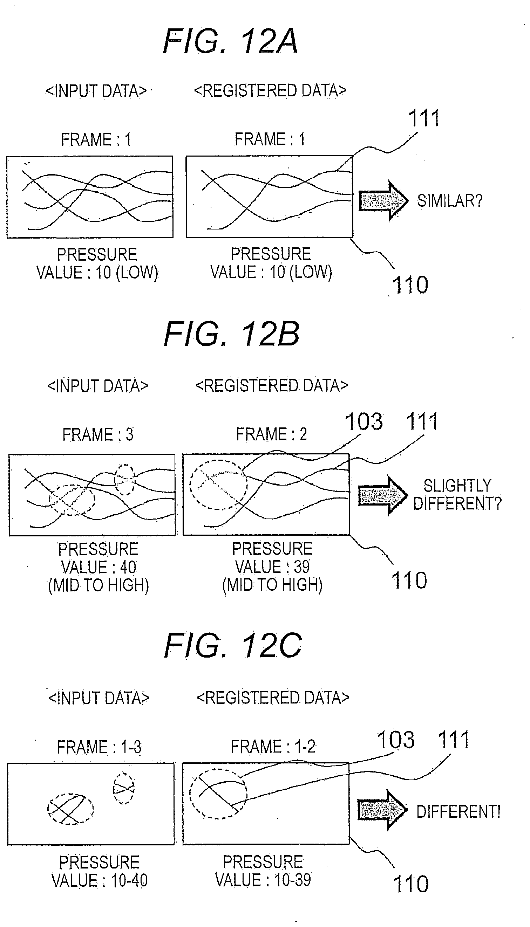

FIGS. 12A, 12B and 12C are explanatory views illustrating a collation process of the registered data and the input data as illustrated in FIGS. 11A and 11B. It is assumed herein that the registered finger and the input finger are different fingers although they are slightly similar to each other, and determining that the fingers are different is the correct result of collation.

In collation, regarding the previously registered pairs of finger images and pressure information, currently input pressure information is observed and compared with the registered pressure information to obtain similar registered pressure vectors. Those similar registered pressure vectors are subjected to collation to compare patterns.

As illustrated in FIG. 11B, the pressure values increase in order of 9, 30, 40, as time to present the finger increases. At this time, for frame 1 having the pressure value 9, data of a similar pressure value 10 is stored in the registered data. If the deviation of the direction of the pressure vector is not more than Th$\theta$, and the deviation of the pressure value is Thp, it is determined then that the pressure vectors are similar, and collation with the registered data is performed. If Thp is 5, for example, the input data having the pressure value 9 and the registered data having the pressure value 10 are subjected to collation. Similarly, the input data having the pressure value 40 and the registered data having the pressure value 38 are subjected to collation.

In the initial collation of input data performed on frame 1, the data having the difference of the pressure value not more than the threshold value Thp is subjected to collation. Therefore, frame 1 of the registered data is subjected to collation, as illustrated in FIG. 12A, and the collation of the frames is performed. It is assumed herein that both patters are relatively similar, but not similar enough to determine that they are identical. A determination standard for such a similarity shall be determined statistically in advance. Image taking still continues, since the authentication processing has not been complete, and frame 2, as illustrated in FIG. 11B, is obtained. It is assumed herein that an image having the pressure value 30 is obtained. Since no data that has the pressure difference not more than Thp against the pressure value 30 exists in the registered data illustrated in FIG. 11A, this registered data is not subjected to collation.

Subsequently, when frame 3 is input, the pressure value is already reaches 40, and the difference of the pressure value between the registered data and the frame 2 image is not more than the threshold value Thp. Therefore, as illustrated in FIG. 12B, the collation between frame 3 of the input data and frame 2 of the registered data is performed. In this processing, the pressed region exists in both frames, which causes the similarity to decrease to some extent compared to the collation result obtained in FIG. 12A. It is assumed that as the similarity is still relatively high, and it cannot be clearly determined in the result that both fingers are different. It is also assumed that both fingers cannot be regarded as the same finger even by combining the collation result of FIG. 12A and the collation result of this time. For example, it is assumed that even if the both results are combined stochastically using Naive Bayes classifier or the like, whether or not the person is a registered person cannot be determined according to the posterior probability. In this case, additional collation is performed.

In further collation, as illustrated in FIG. 12C, the image of frame 1-2 that represents a difference image of the registered data is collated with the image of frame 1-3 that represents a difference image of the input data. The difference images only represent the derived deficiency or modification of the blood vessel pattern caused by pressing. Since these difference images are derived from the images that have actually been used for collation in the past process, the variation of the pressure value is approximately the same for both difference images. Therefore, if the input data and the registered data are of the same finger, and the pressure levels against the finger are similar to each other, then it can be considered that an amount of change is also similar. Meanwhile, if the fingers are different, the three-dimensional shape of the finger, softness of the skin, the thickness of the blood vessel, the concentration of blood, etc. are different. If the pressure value against the finger is changed to a similar level, it can be considered that a larger amount of change of the vein pattern is provided. A large difference may arise if these difference images are collated with each other.

Since the input data and the registered data as illustrated in FIGS. 12A to 12C are of different fingers, it can be seen that the difference image on the input side illustrated in FIG. 12C is largely different from that on the registered side. In particular, there are two pressed regions in the difference image on the input data side, which is a largely different point from the difference image on the registered data side. Accordingly, the collation result of these two images indicates a low similarity. As described above, according to the past collation result, it has not been possible to determine whether the fingers are the same or different. By combining, however, the collation results of the difference images as described above, it is possible to definitely determine whether the fingers are the same or different.

In the above example, it is intended to more clearly determine that the collation result is of different fingers by performing the collation of patterns of the difference frames. Similarly, in the case of collating for the same finger, the likeliness of the same finger can be increased by combining the collation results of the difference frames including similar pressure vectors to obtain a result that the patterns of the difference frames are similar to each other.

As described above, the degree of separation between the same finger and different fingers is thus increased by collating the frames including similar pressure values and also collating the difference images thereof. As a result, the authentication accuracy can be increased.

The images illustrated in FIGS. 11A and 11B, or FIGS. 12A to 12C are different from each other, because the blood vessel has been erased due to the pressing and, as the intensity of the pressing increases, the largeness and the number of pressed regions have changed. Alternatively, the difference may arise by an overall deformation of the pattern by the pressing. In this situation, if the fingers are different, it can also be correctly determined as such, since the degree of deformation may be different when the fingers are different.

Alternatively, the deficiency in the blood vessel pattern due to the pressing can be detected from the difference between frames, and the shape of the region itself may be used for collation. It is commonly known that when the blood is pushed out by pressing a finger, the luminance value at a corresponding part arises. Therefore, when a difference between the image frames that have been photographed sequentially, a luminance difference can be admitted at the pressed location. A particular threshold value may be determined for the luminance difference, and the locations where the luminance has changed higher than the threshold value are converted into data in order to perform collation similarly to the collation of the difference images as described above. Accordingly, even if the blood vessel pattern does not exist at the pressed location, the similarity can be determined regarding the change of the shape of the pressed locations.

Similarly, after the pressed location is detected, a fingerprint pattern or texture of the skin of that location may be used as feature amount for collation. In addition to the shape of the pressed location, that is, the pressed region and the registered pressed region, the information of the skin in such a location can be used to realize even higher collation accuracy.

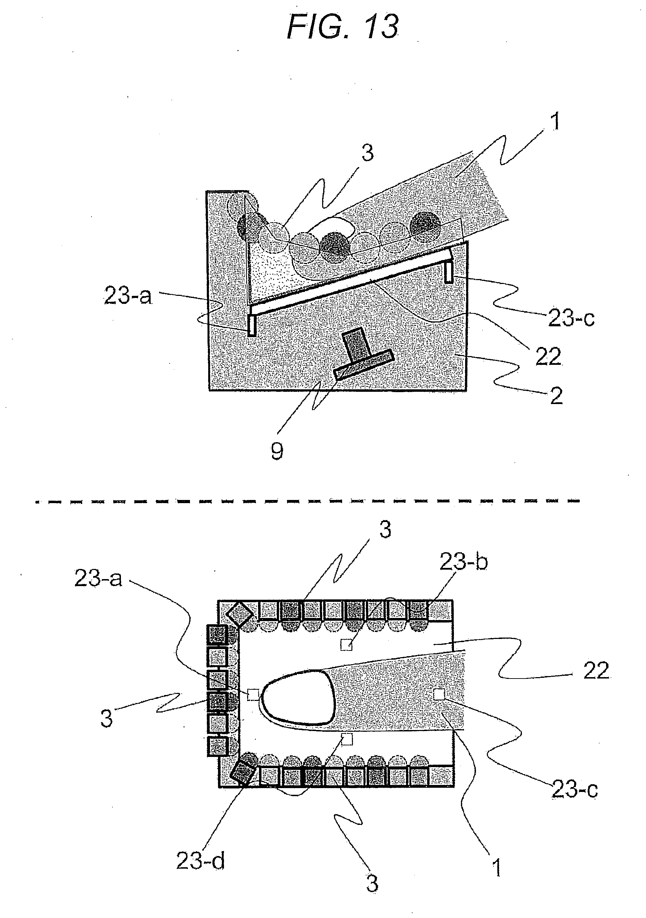

FIG. 13 is a modified example of the above example, where light sources are arranged to surround the finger. In the example illustrated in FIG. 2, the light source has been installed only in front of the finger. In this example, however, many light sources 3 are arranged around the finger. The acrylic plate 22, which defines an opening, is provided larger than the one in the above embodiment, such that an image of the finger can be taken as far as to the vicinity of the first joint of the finger. The light sources, therefore, are also arranged on the side of the finger 1 to irradiate portions of the finger 1 where the light source arranged in front of the finger cannot irradiate. Similar to the above example, the light sources of different wavelengths are arranged alternately to allow light of multiple wavelengths to emit. Pressure sensors 23-a, 23-b, 23-c, and 23-d are provided right below the acrylic plate 22. The acrylic plate 22 is inclined forwards to the front of the finger, and a camera 9 is provided correspondingly in an inclined manner. By providing such an inclination, an effect of facilitating the presentation of the finger 1 horizontally on the acrylic plate 22 and also facilitating the image taking by the camera 9 to take an image of the front of the finger 1. Further, the light sources 3 arranged near the front of the fingertip can irradiate the back side of the finger 1 more easily to obtain a perfect image of transmitted light, leading to a higher contrast of the blood vessel.

When the light sources 3 arranged on the side of the finger emit light to the finger 1, it receives strong light and tends to generate luminance saturation. The light sources can be turned on sequentially to photograph images in time series to synthesize proper portions where the luminance is not saturated. Accordingly, a generally uniform image of the blood vessel can be acquired. This is enabled by using a method according to high dynamic range (HDR) synthesizing or the like.

Images photographed by different light sources that emit light in different directions may include slightly different shadows of the blood vessel image that have been changed depending on the directions of emission. Using this fact, the position and depth of the blood vessel can be estimated. In particular, since the position of the light source is already known, the depth of the blood vessel can be detected according to the principle of triangulation based on the amount of change of the shadow of the blood vessel image and the position of the light source. Slim or thin blood vessels that are hardly seen in the normal state may also be seen clearly at a certain emitting direction or by a light source of a certain wavelength, when various wavelengths of light are directed from multiple directions. Thus, it is possible to synthesize images taken by various illuminating conditions to provide high definition images by HDR synthesizing or super-resolution synthesizing. In the living body, however, light diffuses while proceeding. To cope with this, a model of diffused light components in the living body should be prepared in advance, and this model is combined with deconvolution processing to cancel the diffusion of light. Thus, the images are synthesized while restricting influence of the diffused light, and an accurate structure of high definition blood vessel images can be acquired.

Second Example

In the present example, an exemplary arrangement that is small and capable of improving image processing accuracy of light sources of the above first example will be described.

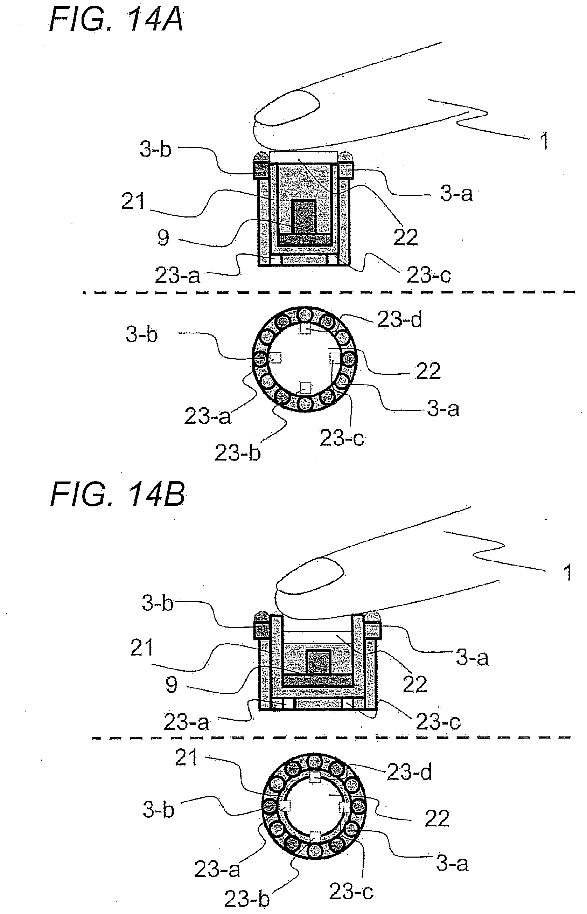

FIGS. 14A and 14B illustrate an example of a small finger vein authentication apparatus including light sources arranged circularly below a finger to be imaged.

As illustrated in the upper view of FIG. 14A, a circular acrylic plate 22 is placed over the opening of the apparatus, and the light sources 3-a, 3-b are arranged circularly around the acrylic plate 22. The light sources of different wavelengths are arranged alternately. Since the light incident position for any wavelength of light source is close, a similar luminance gradient of the image is provided. This is convenient because the occurrence of artifacts by unnecessary differences of luminance can be restricted during processing of synthesizing the images obtained for each wavelength.

Upon presentation of the finger 1 on the acrylic plate 22 by a user, the pressure sensors 23-a, 23-b, 23-c, and 23-d detect the pressure of the finger and the photographing starts to perform authentication.

First, the light source 3-a alone is turned on to take a first image. Subsequently, the light source 3-b alone is turned on to take a second image. Thus, the light source is alternatively turned on one by one until the last light source is turned on to take an image. From these images, the blood vessel image is re-structured and, by using the obtained blood vessel pattern, the removal of wrinkles and blurs, the detection of the depth of the blood vessel, etc. are preformed to thereby performing authentication processing using the correlation between the change of pressure and patterns.

In this example, the contour of the finger cannot be detected, because the opening is not large enough to photograph the finger contour. To cope with this, the positional deviation during collation can be performed by the positional correction using fingerprints. For example, the positional deviation can be corrected by extracting fingerprints using the above described separation technique of wrinkles of the skin, calculating virtual lines running orthogonal to the ridge of the fingerprints at all positions, and regarding a point where all virtual lines are collected as the center position of the fingerprint.

As illustrated in FIG. 14B, the acrylic plate 22 may be placed one step lower so as not to touch the fingertip. This prevents the blood vessel in the entire small region of the fingertip from being lost by the pressure. In particular, since the touch in the small region tends to apply a stronger pressure, there is a constant possibility that the blood vessel patterns may be lost irrespective of whether the pressure is changed. Thus, this tends to reduce the effect of the above described collation method using the relation between the change of pressure and patterns. To cope with this, the finger 1 can be made to touch the camera housing supporting unit 21, instead of the acrylic plate 22, to avoid a large deficiency of the blood vessel. In this structure, the pressure of the finger pressing down the apparatus can be detected, as the pressure sensors being connected to the camera housing supporting unit 21. Indeed, it is expected in this case that the blood vessel patterns are deformed according to the pressing of the finger. By using the correlation of the changes between the pressure values and the blood vessel patterns for the collation, therefore, a still higher accuracy can be realized.

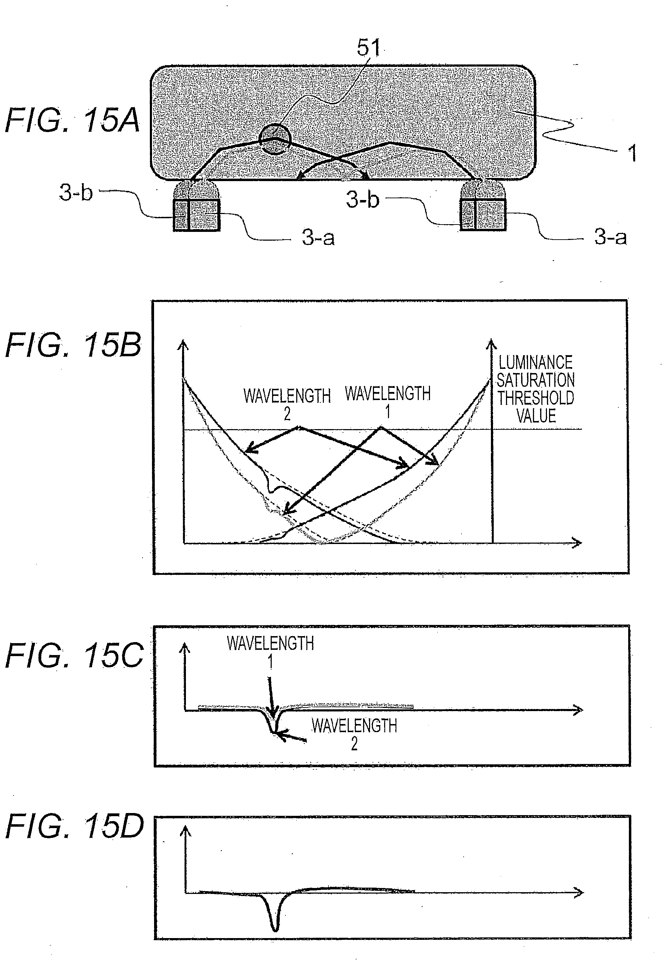

FIGS. 15A, 15B, 15C and 15D illustrate an example of re-structuring the blood vessel image from a plurality of photographed images. First, the light source 3-a emits light to the finger 1. The light scatters while it proceeds into the finger, and attenuates exponentially in the living body according to the distance the light has proceeded. At this time, the light source 3-a is near or in contact with the finger 1 regarded as a subject. The image region close to the light source is overexposed due to luminance saturation. This process is illustrated in the graph of luminance values of FIG. 15B, in which a region exceeding a luminance saturation threshold value is an overexposure part in the image. Since the light attenuates as it proceeds away from the light source, the luminance decreases in a region away from the light source. In this image, therefore, the blood vessel can be observed only at a region located at or more than certain distance away from the light source. A similar trend is provided when the light is emitted from the light source 3-b. The light sources 3-a and 3-b emit light of different wavelengths, and have different optical characteristics for blood, skin, etc. It is assumed that the light source 3-a emits light of a wavelength 1 and the light source 3-b emits light of a wavelength 2, with the wavelength 1 being less reachable and less absorbed in blood.

If no blood vessel exists in the living body, a resulting curve is smooth as illustrated by a dotted line of FIG. 15B. It can be seen that the light reachable range is different for each wavelength, as an attenuation rate of light is different for each wavelength. Meanwhile, since the blood vessel 51 exists in practice, a luminance value slightly decreases, as illustrated by a solid line in this drawing, in the vicinity of the location where the blood vessel exists, due to the influence of light absorption in blood. A shadow appears clearer herein for the wavelength 2, as it has a higher blood absorption ratio.

Therefore, many relations between the distance from the light source and the luminance value when no blood vessel exists can be collected to make data in advance to determine a difference between the data that best fit the luminance curve of the actually observed luminance value. Accordingly, the shadow made by the absorption of the blood vessel alone can be taken out, as illustrated in FIG. 15C.

Such extraction of shadows is performed by lighting a single light source at a time to obtain images. The shadow results of the blood vessel in each image are synthesized by using, for example, a simple addition. Accordingly, highly reliable shadow detection of the blood vessel can be performed. Unobserved regions due to luminance saturation are excluded from the use. This process has been performed as illustrated in FIG. 15D. A difference image of the wavelengths can be enhanced to robustly determine only the shadows caused by the blood vessel. The removal of wrinkles of the skin and blurs may be performed as in the first example.

As a variation of lighting the light sources, a plurality of light sources may be lighted simultaneously, instead of lighting a single light source repeatedly. For example, if the light sources located at point symmetry positions relative to the center of the opening, the blood vessel located in the vicinity of the center of the opening can be clearly irradiated, and the detection accuracy of the blood vessel can be increased. The simultaneous lighting of the plurality of light sources also contributes to high speed photographing. By photographing many images in various emitting patterns and synthesizing the images, the blood vessel images of higher definition can be obtained.

Another method for re-structuring the blood vessel image includes distributing the blood vessels according to a certain assumption, simulating light propagation according to a light propagation equation, determining a difference from the actual image taken, and correcting the assumption according to the difference. This processing can be repeated until the similarity is found between the actual image and the simulated image. Accordingly, the assumed distribution of the blood vessels, by which a certain level of similarity has been given, may be regarded , as an estimation result. Although a lot of time is required for processing, this method allows more correct depth and concentration of the blood vessel to be obtained.

Third Example

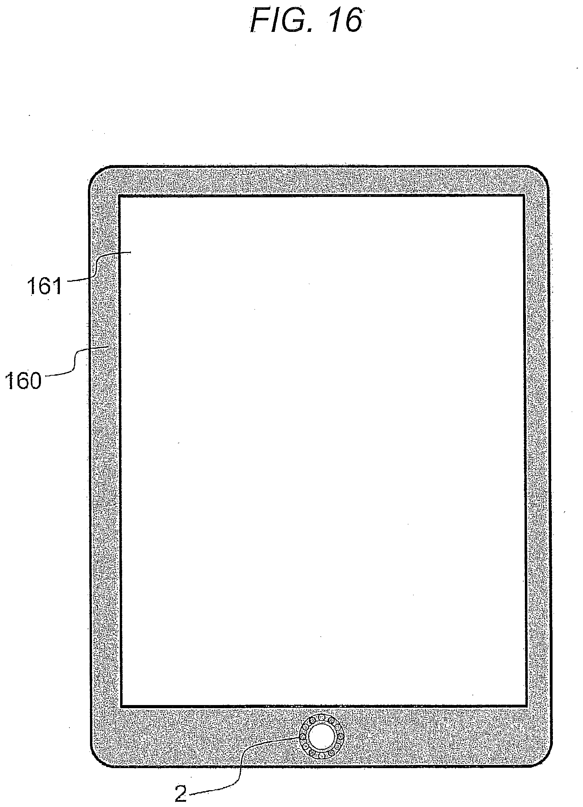

In the present example, an example of a portable terminal to which the authentication apparatus of the second example has been applied is illustrated in FIG. 16. The portable terminal 160 includes a touch panel 161, below which the input device 2 is installed. The input device 2 has a pressure sensor and is able to function as an operation button of the portable terminal. For example, the input device 2 can be pressed down to determine whether the blood vessel matches that of a previously registered user. If a match is found, an automatic login is enabled. Accordingly, the security of the portable terminal can be increased through natural operations.

Other applications include various functions which can be realized by identifying a person, such as identity confirmation for network settlement, retrieval of customized personal settings for each application software, etc. The authentication apparatus is also applicable to amusement software of fortune telling or games, for example, that use a random characteristic of the blood vessel pattern or a collation result of the blood vessel pattern. The authentication apparatus may also be applicable to an on-line social game in which expensive items can be obtained or strong attacking force can be exerted by presenting a finger in a highly reproducible manner. The operation button is not limited to the button shape, and may be formed in any other shape so long as it functions as an operation unit of sensors or the like.

Fourth Example

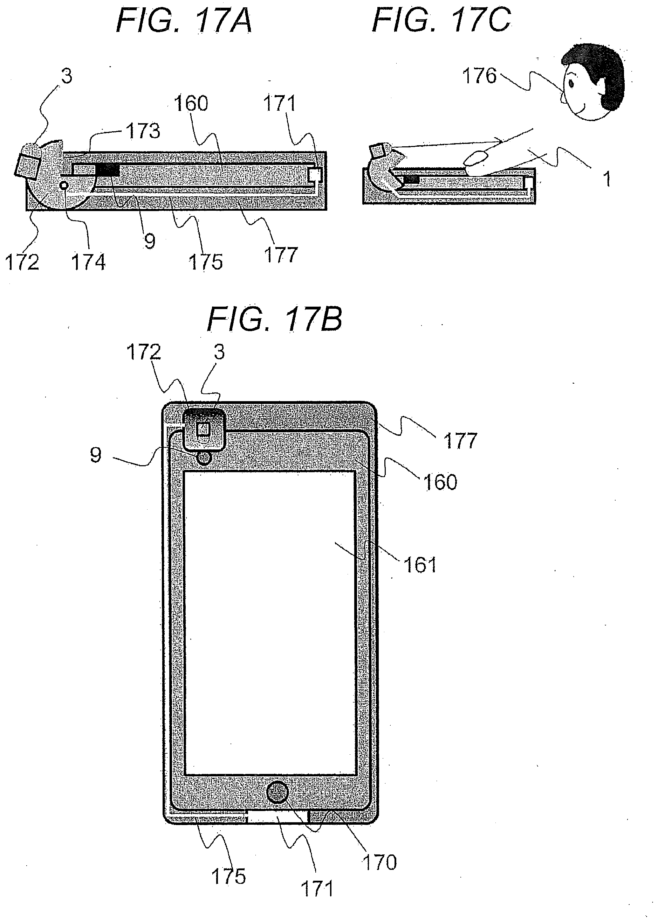

FIGS. 17A to 17C illustrate an example of the biometric authentication apparatus to which the structure of other examples is applied. The biometric authentication apparatus includes an imaging device which is essentially installed in a portable terminal and includes a mirror and a light source provided in a protection case of the terminal such as a smartphone or tablet.

A portable terminal 160, such as a smartphone or a tablet terminal, includes the camera 9 that is installed in the portable terminal 160. In general, the touch panel 161 is located in the center of the device, and a button 170 used for various operations is installed on the side or under the front surface of the device. Further, an interface connector 171 for use in communications with external devices and charging may be provided. The small camera 9 is installed on the upper part of the front or back of the device.

As illustrated in FIGS. 17A and 17B, in the example, a rotary unit 172 capable of moving to a position within the field of view of the camera 9 installed on the upper part of the device is provided in a protection case 177. A mirror 173 and a rotation detecting switch 174 are attached to the rotary unit 172, and the rotary unit 172 can detect the rotating state of the rotary unit 172 by the rotation detecting switch 174. The rotary unit 172 includes a light source 3 in which three LEDs capable of emitting red, green, and blue wavelengths of visible light are installed. Power is supplied to the light source via a signal cable 175 that connects the interface connector 171 and the rotary unit 172. The signal cable 175 is also used to communicate with a processing device in the portable terminal to advise the state of the rotation detecting switch 174.

The user rotates the rotary unit 172 with a finger towards the front side of the apparatus when the personal authentication is needed for login to the terminal, network settlement, etc. At this time, as illustrated in FIG. 17C, the mirror 173 covers and hides a portion of the upper part of the camera 9 to allow it to take an image, via the mirror 173, of the finger that is in contact with the touch panel 161. The light source 3 can also irradiate the finger 1. A portion that is not covered by the mirror 173 can normally image surroundings and reflect a face 176 of the user or the like in this portion. When the rotary unit 172 is rotated by the user, the rotation of the mirror is communicated to the processing device in the portable terminal 160 via the signal cable 175 and the interface connector 171. Upon detection of this state, the biometric authentication starts.

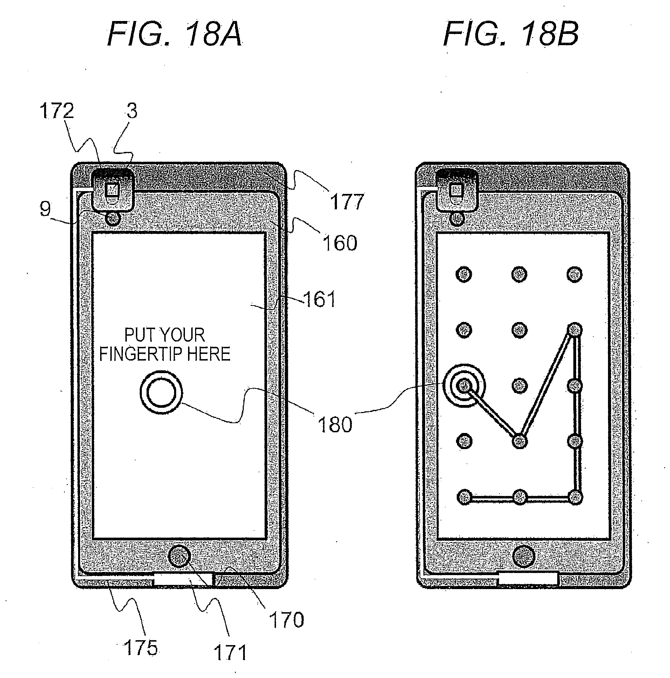

FIGS. 18A and 18B illustrate an example of guidance of the finger displayed on the touch panel. As illustrated in FIG. 18A, the user can present the finger on a proper position by matching the fingertip to a fingertip position mark 180 of the guidance. Upon detection of putting the finger on the proper position, the three LEDs of the light source 3 start emitting light alternately to the finger, and three visible light images of the three wavelengths are obtained. Based on these three images, the blood vessel portion alone may be enhanced according to the relation of blood absorption ratio, or only the blood vessel portion is acquired according to a stochastic method such as independent component analysis.

Since the touch panel 161 can detect the touching position of the finger, it is possible to provide guidance of positional deviation from a fingertip position mark 180, or otherwise start the authentication so long as the finger is put within an allowable range, even though the finger is not put on the fingertip position mark 180. The fingertip position mark 180 can be moved dynamically to image the finger at various angles. By registering these images, the authentication can be performed even when the position of the finger is changed. At this time, the light source 3 can have a wide angle directivity, and the finger can be irradiated uniformly even when the finger is placed at any position.

As illustrated in FIG. 18B, a line pattern, which has been widely performed in current smartphones or the like, can be input. By using a combination of inputting a password, which increases security, and presentation of the finger of this example, an additionally strong security can be realized. Specifically, the finger image can photographed from various angles by moving the finger, additional information of the finger structure can be obtained to increase authentication accuracy. For example, a technique for measuring three-dimensional shape, such as the one represented by structure from motion (SFM), may be applied. Accordingly, such an effect that the three-dimensional structure of the finger can be estimated to increase the amount of usable information in the biometric authentication, or the images of the finger of various angles can be registered to increase an allowable limit of the positional deviation of the finger even when the presented finger position is deviated, can be expected.

Similarly, the authentication can be performed by placing the finger on an arbitrary position without displaying the fingertip position mark 180. Since the finger position can be detected from the touch panel 161, the registered finger position and the input finger position can be normalized to match each other by deformation such as enlargement and reducing of the images. Accordingly, the user can present the finger more conveniently while restricting the degradation of authentication accuracy.

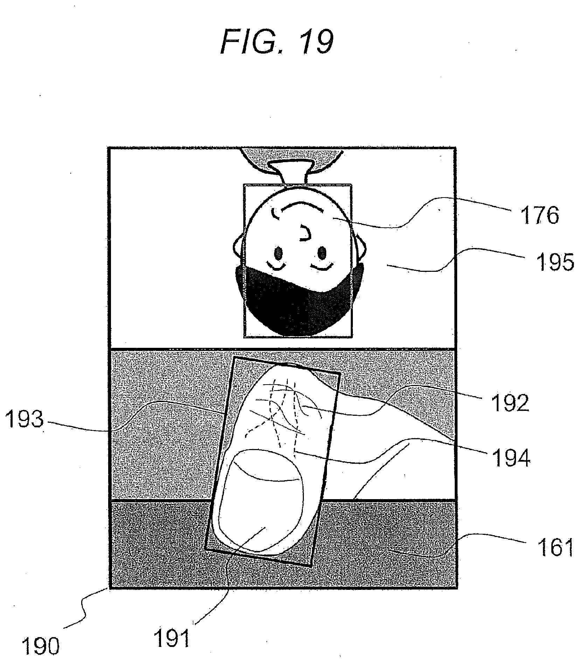

FIG. 19 illustrates an example of the face and the finger of the user reflected on the mirror during the image taking.

A finger image 190 presented by the user includes various types of information, such as a nail 191, wrinkles 192 of the finger joint, a color of the finger 1, the size and position 193 of the finger 1, and a finger vein pattern 194. As described above, the mirror 173 only covers about a half of the upper part of the camera 9, and the front side of the portable terminal is reflected by the mirror 173 and projected in the part of the image. In the other part of the region, background 195 that can be imaged directly by the camera 9 and the face 176 of the user are projected in a vertically reverse manner. These elements are extracted by a common image processing method to determine a similarity for each element, and the collation results are lastly synthesized to calculate the similarity between the registered finger and the input finger.

In the above example, the finger is irradiated by the light source, but the light source 3 may be deleted and surrounding visible light can be used instead. In this case, power consumption is lowered, although there is some restriction for the use in a low brightness place, leading to cost reduction.

In the above example, the mirror and the light source are provided on the portable terminal case, but the similar effect can be obtained by tilting the optical axis of the front camera such that it can take an image near the touch panel. The similar effect can also be obtained by providing the light source on the portable terminal. In the case of separately installing the front camera with a tilted optical axis, if there is a problem of installation in cost or space, the front camera with the tilted optical axis may be provided. In this case, a slide switch, for example, is provided to tilt the optical switch such that the optical axis of a normal front camera is mechanically or electrically tilted by operating the slide switch. Accordingly, the upper part of the touch panel can be included in the field angle and the authentication processing can start. Thus, mostly the similar function to that of the above examples can be obtained without providing more than one front cameras.

Features, components and specific details of the structures of the above-described examples and embodiments may be exchanged or combined to form further configurations optimized for the respective application. As far as those modifications are readily apparent for an expert skilled in the art they shall be disclosed implicitly by the above description without specifying explicitly every possible combination, for the sake of conciseness of the present description.

Industrial Applicability

A small, highly convenient, and highly accurate biometric authentication apparatus can be realized. Such an apparatus is useful as a personal authentication apparatus.

Reference Signs List

1 finger
2 input device
3 light source
9 camera
10 authentication processing unit
11 central processing unit
12 memory
13 interface
14 storage device
15 display unit
16 input unit
17 speaker
18 image input unit
21 camera housing supporting unit
22 acrylic plate
23 pressure sensor
41 structuring element
42 shadow of wrinkles
43 wrinkle component
44 shadow of blood vessel
51 blood vessel
52 dermis
53 wrinkles of skin
80 projected two dimensional blood vessel image
81 blood vessel pattern
82 blood vessel pattern
83 three-dimensional blood vessel image
100 finger image
101 finger contour
102 fingertip
103 pressed region
104 pressure vector
110 collation data
111 blood vessel pattern
160 portable terminal
170 operation button
171 interface connector
172 rotary unit
173 mirror
174 rotation detecting switch
175 signal cable
176 face
177 protective
180 fingerprint position mark
190 finger image
191 nail
192 wrinkles at finger joint

193 size and positional information
194 finger vein pattern
195 background

**Claims**

1. A blood vessel image capturing apparatus, comprising:

a position area on which a finger (1) can be placed;
a light source (3) configured to irradiate the finger (1) presented on the position area;
an imaging unit (9) configured to form an image of light emitted from the light source (3) and transmitted through the finger (1);
a pressure detecting unit (23) with at least four pressure sensors (23-a, 23-b, 23-c, 23-d) provided at four locations, configured to detect pressure of the finger (1) placed on the position area; and
an image processing unit (10) configured to:

process an image formed by the imaging unit (9),
extract a finger contour (101), and
obtain a position of a pressed region (103) where the pressure of the finger (1) is at or more than a predetermined pressure value, wherein the image processing unit (10) is further configured to:

determine a pressure vector (104) which is defined as having a direction towards the center of the pressed region (103) that is determined according to a ratio of the pressure values of the four pressure sensors (23-a, 23-b, 23-c, 23-d),
calculate a similarity between the pressure vector (104)and a registered pressure vector determined from each of previously registered plural images, and
select, according to the calculated similarity, a registered image of the registered plural images to be collated with the image.

2. The blood vessel image capturing apparatus according to claim 1, **characterized by**
further comprising a housing,
the imaging unit (9) arranged at the bottom of the housing, and
the at least four pressure detecting units (23-a, 23-b, 23-c, 23-d) arranged at the bottom of the housing.

3. The blood vessel image capturing apparatus according to any one of the preceding claims, **characterized in that**
the light source (3) is configured to emit light of a first wavelength and light of a second wavelength different from the first wavelength,
the imaging unit (9) is configured to photograph a first image by the light of the first wavelength and a second image by the light of the second wavelength,
the image processing unit (10)
is configured to compare a luminance value of the first image with a luminance value of the second image,
to separate information of a blood vessel region of a blood vessel image,
to generate, by using the information of the blood vessel region, a blood-vessel-removed image formed by removing the information of the blood vessel region from the first image,
to extract noise information included in the blood-vessel-removed image, and
to remove, by using the noise information, the noise information from the first image to obtain a blood vessel image for authentication.

4. The blood vessel image capturing apparatus according to claim 3, **characterized in that**
the blood vessel image for authentication and the noise information are collated with previously registered data.

5. The blood vessel image capturing apparatus according to claim 3 or 4, **characterized in that**
the light of the second wavelength has a light absorption ratio in blood smaller than that of the light of the second wavelength, and
the image processing unit (10)
is configured to compare the blood vessel image for authentication with the second image, and
to calculate a three-dimensional shape of a blood vessel pattern included in the blood vessel image for authentication.

6. The blood vessel image capturing apparatus according to any one of the preceding claims, **characterized in that** the plural registered images have previously been stored with registered pressure values corresponding to each of the registered images, and
the image processing unit (10)
is configured to detect a registered pressure value of the registered plural pressure values corresponding to the pressure value detected by the pressure detecting unit (23), and
to calculate a similarity between the positions of the pressed region (103) and the pressed region included in the registered image corresponding to the detected registered pressure value.

7. The blood vessel image capturing apparatus according to any one of the preceding claims, **characterized in that** the light source (3) includes
plural first light sources configured to emit the light of the first wavelength, and plural second light sources configured to emit the second wavelength,
the plural first and second light sources being arranged circularly around the position area.

8. The blood vessel image capturing apparatus according to claim 7, **characterized in that** the plural first and second light sources are arranged alternately.


**Patentansprüche**

1. Blutgefäßbildaufnahmevorrichtung, die Folgendes umfasst:

    einen Positionsbereich, auf den ein Finger (1) gelegt werden kann;
    eine Lichtquelle (3), die konfiguriert ist, den Finger (1), der auf dem Positionsbereich gezeigt ist, zu bestrahlen;
    eine Abbildungseinheit (9), die konfiguriert ist, ein Bild aus von der Lichtquelle (3) ausgesendetem und durch den Finger (1) durchgelassenem Licht zu erzeugen;
    eine Druckdetektionseinheit (23) mit mindestens vier Drucksensoren (23-a, 23-b, 23-c, 23-d), die an vier Orten vorgesehen sind, die konfiguriert ist, den Druck des auf den Positionsbereich gelegten Fingers (1) zu detektieren; und
    eine Bildverarbeitungseinheit (10), die konfiguriert ist:

        ein durch die Abbildungseinheit (9) erzeugtes Bild zu verarbeiten,
        einen Fingerumriss (101) zu extrahieren und
        eine Position eines gedrückten Bereichs (103), wo der Druck des Fingers (1) bei einem vorgegeben Druckwert oder mehr als diesem liegt, zu erhalten, wobei
        die Bildverarbeitungseinheit (10) ferner konfiguriert ist:

            einen Druckvektor (104) zu bestimmen, der so definiert ist, dass er in Richtung des Mittelpunkts des gedrückten Bereichs (103) orientiert ist und der gemäß einem Verhältnis der Druckwerte der vier Drucksensoren (23-a, 23-b, 23-c, 23-d) bestimmt wird,
            eine Ähnlichkeit zwischen dem Druckvektor (104) und einem registrierten Druckvektor, der von jedem von vorher registrierten mehreren Bildern bestimmt wurde, zu berechnen und
            gemäß der berechneten Ähnlichkeit ein registriertes Bild der registrierten mehreren Bilder auszuwählen, um es mit dem Bild zu vereinigen.

2. Blutgefäßbildaufnahmevorrichtung nach Anspruch 1, **dadurch gekennzeichnet, dass** sie ferner ein Gehäuse umfasst,
die Abbildungseinheit (9) an der Unterseite des Gehäuses angeordnet ist und
die mindestens vier Druckdetektionseinheiten (23-a, 23-b, 23-c, 23-d) an der Unterseite des Gehäuses angeordnet sind.

3. Blutgefäßbildaufnahmevorrichtung nach einem der vorhergehenden Ansprüche, **dadurch gekennzeichnet, dass** die Lichtquelle (3) konfiguriert ist, Licht einer ersten Wellenlänge und Licht einer zweiten Wellenlänge, die von der ersten Wellenlänge verschieden ist, auszusenden,
die Abbildungseinheit (9) konfiguriert ist, ein erstes Bild durch das Licht der ersten Wellenlänge und ein zweites Bild durch das Licht der zweiten Wellenlänge zu fotografieren,
die Bildverarbeitungseinheit (10) konfiguriert ist, einen Helligkeitswert des ersten Bildes mit einem Helligkeitswert des zweiten Bildes zu vergleichen,

Informationen eines Blutgefäßbereichs eines Blutgefäßbildes abzusondern, durch Verwenden der Informationen des Blutgefäßbereichs ein Bild mit entfernten Blutgefäßen zu erzeugen, das durch Entfernen der Informationen des Blutgefäßbereichs aus dem ersten Bild erzeugt wird,

Rauschinformationen, die in dem Bild mit entfernten Blutgefäßen enthalten sind, zu extrahieren und

durch Verwenden der Rauschinformationen die Rauschinformationen aus dem ersten Bild zu entfernen, um ein Blutgefäßbild für eine Authentifizierung zu erhalten.

**4.** Blutgefäßbildaufnahmevorrichtung nach Anspruch 3, **dadurch gekennzeichnet, dass**

das Blutgefäßbild für eine Authentifizierung und die Rauschinformationen mit vorher registrierten Daten vereinigt werden.

**5.** Blutgefäßbildaufnahmevorrichtung nach Anspruch 3 oder 4,
**dadurch gekennzeichnet, dass**

das Licht der zweiten Wellenlänge ein Lichtabsorptionsverhältnis von Blut besitzt, das kleiner als das des Lichts der zweiten Wellenlänge ist, und

die Bildverarbeitungseinheit (10) konfiguriert ist, das Blutgefäßbild für eine Authentifizierung mit dem zweiten Bild zu vergleichen und

eine dreidimensionale Form eines Blutgefäßmusters, das in dem Blutgefäßbild für eine Authentifizierung enthalten ist, zu berechnen.

**6.** Blutgefäßbildaufnahmevorrichtung nach einem der vorhergehenden Ansprüche, **dadurch gekennzeichnet, dass**

die mehreren registrierten Bilder vorher mit registrierten Druckwerten gespeichert wurden, die jedem der registrierten Bilder entsprechen, und

die Bildverarbeitungseinheit (10) konfiguriert ist, einen registrierten Druckwert der registrierten mehreren Druckwerte, der dem durch die Druckdetektionseinheit (23) detektierten Druckwert entspricht, zu detektieren und

eine Ähnlichkeit zwischen den Positionen des gedrückten Bereichs (103) und dem in dem registrierten Bild enthaltenen gedrückten Bereich, der dem detektierten registrierten Druckwert entspricht, zu berechnen.

**7.** Blutgefäßbildaufnahmevorrichtung nach einem der vorhergehenden Ansprüche, **dadurch gekennzeichnet, dass**
die Lichtquelle (3) enthält:

mehrere erste Lichtquellen, die konfiguriert sind, das Licht der ersten Wellenlänge auszusenden, und mehrere zweite Lichtquellen, die konfiguriert sind, die zweite Wellenlänge auszusenden,

wobei die mehreren ersten und zweiten Lichtquellen kreisförmig um den Positionsbereich angeordnet sind.

**8.** Blutgefäßbildaufnahmevorrichtung nach Anspruch 7, **dadurch gekennzeichnet, dass**
die mehreren ersten und zweiten Lichtquellen abwechselnd angeordnet sind.

**Revendications**

**1.** Appareil de capture d'image de vaisseau sanguin, comprenant :

une zone de position sur laquelle un doigt (1) peut être placé ;
une source de lumière (3) configurée pour irradier un doigt (1) présenté sur la zone de position ;
une unité d'imagerie (9) configurée pour former une image de la lumière émise par la source de lumière (3) et transmise à travers le doigt (1) ;
une unité de détection de pression (23) avec au moins quatre capteurs de pression (23-a, 23-b, 23-c, 23-d) prévus à quatre emplacements, configurée pour détecter la pression du doigt (1) placé sur la zone de position ; et
une unité de traitement d'image (10) configurée pour :

traiter une image formée par l'unité d'imagerie (9),
extraire un contour de doigt (101) et
obtenir une position d'une région pressée (103) où la pression du doigt (1) est supérieure ou égale à une valeur de pression prédéterminée,
dans lequel
l'unité de traitement d'image (10) est en outre configurée pour :

déterminer un vecteur de pression (104) qui est défini comme ayant une direction vers le centre de la région pressée (103) qui est déterminée en fonction d'un rapport des valeurs de pression des quatre capteurs de pression (23-a, 23-b, 23-c, 23-d),

calculer une similarité entre le vecteur de pression (104) et un vecteur de pression enregistré déterminé à partir de chacune des plusieurs images précédemment enregistrées et

sélectionner, en fonction de la similarité calculée, une image enregistrée desdites plusieurs images enregistrées à assembler avec l'image.

2.  Appareil de capture d'image de vaisseau sanguin selon la revendication 1, **caractérisé en ce qu'**il comprend en outre un boîtier,

l'unité d'imagerie (9) étant agencée sur le fond du boîtier et

lesdites au moins quatre unités de détection de pression (23-a, 23-b, 23-c, 23-d) étant agencées sur le fond du boîtier.

3.  Appareil de capture d'image de vaisseau sanguin selon l'une quelconque des revendications précédentes, **caractérisé en ce que**

la source de lumière (3) est configurée pour émettre une lumière d'une première longueur d'onde et une lumière d'une seconde longueur d'onde différente de la première longueur d'onde,

l'unité d'imagerie (9) est configurée pour photographier une première image par la lumière de la première longueur d'onde et une seconde image par la lumière de la seconde longueur d'onde,

l'unité de traitement d'image (10)

est configurée pour comparer une valeur de luminance de la première image avec une valeur de luminance de la seconde image,

pour séparer les informations d'une région de vaisseau sanguin d'une image de vaisseau sanguin,

pour générer, en utilisant les informations de la région de vaisseau sanguin, une image débarrassée du vaisseau sanguin formée en éliminant les informations de la région de vaisseau sanguin de la première image,

pour extraire les informations de bruit incluses dans l'image débarrassée du vaisseau sanguin, et

pour éliminer, en utilisant les informations de bruit, les informations de bruit de la première image pour obtenir une image de vaisseau sanguin pour authentification.

4.  Appareil de capture d'image de vaisseau sanguin selon la revendication 3, **caractérisé en ce que**

l'image de vaisseau sanguin pour authentification et les informations de bruit sont assemblées avec les données précédemment enregistrées.

5.  Appareil de capture d'image de vaisseau sanguin selon la revendication 3 ou 4, **caractérisé en ce que**

la lumière de la seconde longueur d'onde a un taux d'absorption de la lumière dans le sang inférieur à celui de la lumière de la seconde longueur d'onde et

l'unité de traitement d'image (10)

est configurée pour comparer l'image de vaisseau sanguin pour authentification avec la seconde image et

pour calculer une forme tridimensionnelle d'un motif de vaisseau sanguin inclus dans l'image de vaisseau sanguin pour authentification.

6.  Appareil de capture d'image de vaisseau sanguin selon l'une quelconque des revendications précédentes, **caractérisé en ce que**

lesdites plusieurs images enregistrées ont préalablement été stockées avec des valeurs de pression enregistrées correspondant à chacune des images enregistrées et

l'unité de traitement d'image (10)

est configurée pour détecter une valeur de pression enregistrée parmi lesdites plusieurs valeurs de pression enregistrées correspondant à la valeur de pression détectée par l'unité de détection de pression (23) et

pour calculer une similarité entre les positions de la région pressée (103) et de la région pressée incluse dans l'image enregistrée correspondant à la valeur de pression enregistrée détectée.

7.  Appareil de capture d'image de vaisseau sanguin selon l'une quelconque des revendications précédentes, **caractérisé en ce que**

la source de lumière (3) inclut

plusieurs premières sources de lumière configurées pour émettre la lumière de la première longueur d'onde et plusieurs secondes sources de lumière configurées pour émettre la seconde longueur d'onde,

lesdites plusieurs premières et secondes sources de lumière étant agencées circulairement autour de la zone de position.

8. Appareil de capture d'image de vaisseau sanguin selon la revendication 7, **caractérisé en ce que** lesdites plusieurs premières et secondes sources de lumière sont agencées alternativement.

# FIG. 1

# FIG. 2

# FIG. 3

START

S301　NUMBER OF FRAMES F = 0, DETECT PRESSURE VALUE P FROM PRESSURE SENSOR

S302　IS PRESSURE APPLIED, OR IS FINGER DETECTED?

Y　　　N

S303　UPDATE NUMBER OF FRAMES: F+1 → F

S304　TAKE IMAGE BY EMITTING INFRARED LIGHT FROM LIGHT SOURCE 3-C

S305　DETECT FINGER CONTOUR

S306　ADJUST LIGHT AMOUNT OF LIGHT SOURCES 3-a, 3-b, 3-c, AND ACQUIRE 3-WAVELENGTHS IMAGE

S307　ACQUIRE PRESSURE VALUE

S308　FEATURE EXTRACTING PROCESSING (POSITIONAL CORRECTION, REMOVAL OF WRINKLES OF SKIN AND BLURS, DETECTION OF BLOOD VESSEL PATTERN AND DEPTH)

S309　PRESSURE VECTOR → P'

S310　COLLATION WITH REGISTERED PATTERN SIMILAR TO P'

S311　MATCH REGISTERED PATTERN?

N　　　Y

S312　CALCULATE DIFFERENCE BETWEEN FRAMES 1 TO F-1 AND FRAME F

S313　COLLATION WITH DIFFERENCE OF REGISTERED PATTERN HAVING SIMILAR PRESSURE VECTOR P'

S314　MATCH REGISTERED PATTERN?

N　　　Y

S315　DETERMINE STOP CONDITION

S316　IS STOP CONDITION SATISFIED?

N　　　Y

S317　AUTHENTICATION FAILED

S318　AUTHENTICATION SUCCEEDED

END　　　END

# FIG. 4A

# FIG. 4B

# FIG. 5

## FIG. 6A

IMAGE TAKING OF 2-
WAVELENGTH IMAGE

LUMINANCE VALUE

$g_1$

$g_2$

44

44 42 42

42

## FIG. 6B

REMOVAL OF BLOOD
VESSEL PORTION

LUMINANCE INCREASED
(if $g_1 > g_2$)

## FIG. 6C

Top-hat CONVERSION

41

42 42

42

## FIG. 6D

REMOVAL OF WRINKLE
PORTION

RESULT

## FIG. 7

## FIG. 8A

## FIG. 8B

RELATIVE DEPTH

# FIG. 9

## FIG. 10A

## FIG. 10B

## FIG. 10C

# FIG. 11A

<REGISTERED DATA>

FRAME : 1

PRESSURE
VALUE : 10 (LOW)

FRAME : 2

PRESSURE
VALUE : 38 (MID TO HIGH)

FRAME : 3

PRESSURE
VALUE : 56 (HIGH)

FRAME : 1-2

PRESSURE
VALUE : 10-38

FRAME : 2-3

PRESSURE
VALUE : 38-56

FRAME : 1-3

PRESSURE
VALUE : 10-56

# FIG. 11B

<INPUT DATA>

FRAME : 1

PRESSURE
VALUE : 9 (LOW)

FRAME : 2

PRESSURE
VALUE : 30 (MID)

FRAME : 3

PRESSURE
VALUE : 40 (MID TO HIGH)

FRAME : 1-2

PRESSURE
VALUE : 9-30

FRAME : 2-3

PRESSURE
VALUE : 30-40

FRAME : 1-3

PRESSURE
VALUE : 9-40

## FIG. 12A

<INPUT DATA>            <REGISTERED DATA>

FRAME : 1              FRAME : 1

PRESSURE              PRESSURE
VALUE : 10 (LOW)       VALUE : 10 (LOW)

SIMILAR?

111

110

## FIG. 12B

<INPUT DATA>            <REGISTERED DATA>

FRAME : 3              FRAME : 2

PRESSURE              PRESSURE
VALUE : 40            VALUE : 39
(MID TO HIGH)         (MID TO HIGH)

SLIGHTLY
DIFFERENT?

103  111

110

## FIG. 12C

<INPUT DATA>            <REGISTERED DATA>

FRAME : 1-3           FRAME : 1-2

PRESSURE             PRESSURE
VALUE : 10-40         VALUE : 10-39

DIFFERENT!

103  111

110

# FIG. 13

FIG. 14A

FIG. 14B

FIG. 15A

51

1

3-b

3-a

3-b

3-a

FIG. 15B

WAVELENGTH 2    WAVELENGTH 1

LUMINANCE SATURATION THRESHOLD VALUE

FIG. 15C

WAVELENGTH 1

WAVELENGTH 2

FIG. 15D

# FIG. 16

## FIG. 17A

3

173   160   171

172   174   9   175   177

## FIG. 17C

176

1

## FIG. 17B

172   3

9

177

160

161

175   171   170

# FIG. 18A

# FIG. 18B

PUT YOUR
FINGERTIP HERE

# FIG. 19

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011194245 A

- EP 2312495 A